# EUROPEAN PATENT APPLICATION

(11) **EP 1 627 925 A1**
(43) Date of publication of application: **22.02.2006**
(21) Application number: 05007423.6
(22) Date of filing: 05.04.2005
(51) Int. Cl.: C12Q 1/68, C12N 15/11

(54) **Method for the determination of cellular transcriptional regulation by micro RNAs**

(30) Priority: 06.04.2004 US 818956
(71) Applicant: Eppendorf Array Technologies, 5000 Namur (BE)
(72) Inventor: Van Huffel, Christophe, 5000 Namur (BE); Remacle, José, 5020 Malonne (BE); Bülow, Sven, 22395 Hamburg (DE); Zammatteo, Nathalie, 5024 Gelbressée (BE)
(74) Representative: Becker Kurig Straus

(57) **Abstract**

The present invention relates to a new method for determining the RNAi mediated transcriptional regulation of a cell by the determination of a pattern of at least 3 miRNA detected simultaneously and quantified in the same cell extract. The determination of a pattern of miRNA comprises the steps of: (i) providing an array onto which are fixed capture probes, said capture probes being arranged on pre-determined locations and reflecting the genomic or transcriptional matter of a cell; (ii) isolating a miRNA pool potentially present from a cell; (iii) elongating or ligating said miRNAs into labeled capture probes, (iv) contacting said labeled polynucleotides with the array under conditions allowing hybridization of the labeled polynucleotides to complementary capture probes present on the array; (v) detecting and quantifying a signal present on the specific locations of the array, wherein the detection of a pattern of at least 3 signals on the array reflects the pattern of miRNAs being involved in the RNAi mediated cellular transcriptional regulation.

## Description

### Field of the invention

The present invention relates to the field of interference RNA (RNAi). In particular, the present invention relates to a method for the determination of the cellular transcriptional regulation based on a simultaneous detection and quantification of a pattern of miRNAs in a cell.

### Description of the related art

In experiments, during which dsRNA was injected into the nematode *Caenorhabditis elegans* it was found that a silencing of genes highly homologous in sequence to the delivered dsRNA occurred (Fire et al., Nature **391** (1998), 806-811). Based on this finding the term "RNA interference" (RNAi) was created nominating the capability of such dsRNA-molecules to affect the translation of transcripts.

During ensuing research in this area it has been shown that dsRNA triggers degradation of homologous RNAs within the region of identity with the dsRNA (Zamore et al., Cell 101 (2000), 25-33). Apparently, the dsRNA is processed to RNA fragments exhibiting a length of about 21-23-ribonucleotides (Zamore et al., Cell **101** (2000), 25-33). These short fragments were also detected in extracts prepared from *Drosophila melanogaster* Schneider 2 cells that were transfected with dsRNA before cell lysis (Hammond et al., Nature **404** (2000) 293-296) or after injection of radiolabeled dsRNA into *D. melanogaster* embryos (Yang et al., Curr. Biol. **10** (2000) 1191-1200) or *C*. *elegans* adults (Parrish et al., Mol. Cell **6** (2000), 1077-1087).

RNAi was observed to also be naturally present in a wide range of living cells. E.g. these kind of molecules has been found to exist in insects (Kennerdell and Carthew, Cell **95** (1998), 1017-1026), frog (Oelgeschlager et al., Nature **405** (2000), 757-763), and other animals including mice (Svoboda et al., Development **127** (2000), 4147-4156; Wianny and Zernicka-Goetz, Nat. Cell Biol. **2** (2000), 70-75) and also in humans. RNA molecules of similar size have also been found to accumulate in plant tissue that exhibits post-transcriptional gene-silencing (PTGS) (Hamilton and Baulcombe, Sciences **286** (1999), 950-952).

The natural function of RNAi and co-suppression appears to be the protection of the genome against invasion by mobile genetic elements, such as transposons and viruses, which produce aberrant RNA or dsRNA in the host cell when they become active (Jensen et al., Nat. Genet. **21** (1999), 209-212; Ketting et al., Cell **99** (1999), 133-141; Ratcliff et al., Plant Cell **11** (1999), 1207-1216; Tabara et al., Cell **99** (1999), 123-132; Malinsky et al., Genetics **156** (2000), 1147-1155). Specific mRNA degradation prevents transposon and virus replication, although some viruses seem to be able to overcome or prevent this process by expressing proteins that suppress PTGS (Anandalakshmi et al., Science **290** (2000), 142-144; Lucy et al., EMBO J. **19** (2000), 1672-1680; Voinnet et al., Cell **103** (2000), 153-167).

The currently existing model for the mechanism of RNAi is based on the observation that the introduced dsRNA is bound and cleaved by RNase III-like enzyme Dicer to generate products having the length indicated above. These molecules, termed small interfering RNAs (siRNAs) trigger the formation of RNA-induced silencing complex (RISC). The resulting dsRNA-protein complexes appear to represent the active effectors of selective degradation of homologous mRNA (Hamilton and Baulcombe, Science **286** (1999), 950-952, Zamore et al., Cell **101** (2000), 25-33; Elbashir et al., Genes & Dev. **15** (2001), 188-200. Elbashir et al. provide evidence that the direction of dsRNA processing determines, whether sense or antisense target RNA can be cleaved by the siRNA-protein complex. Helicases in the complex unwind the dsRNA, and the resulting single-stranded RNA (ssRNA) seems to be used as a guide for substrate selection. Once the ssRNA is base-paired with the target mRNA, a nuclease activity, presumably within the complex, degrades the mRNA.

The enzymatic machinery for generating siRNA also appears to be used for the production of a second class of endogenously encoded, small RNA molecules termed microRNAs (miRNAs). miRNAs regulate mRNA translation whereas siRNAs direct RNA destruction via the RNA interference pathway. miRNAs are processed through at least two sequential steps; generation of 70 nucleotides (pre-miRNAs) from the longer transcripts (termed pri-miRNAs) and processing of pre-miRNAs into mature miRNAs (Lee et al. Embo **J. 21** (2002), 4663-4670). miRNAs are typically 20-22 nucleotides non coding RNA that regulate expression of mRNA exhibiting sequences complementary thereto. They are numerous and widespread among eukaryotes, being conserved throughout evolution.

Research on miRNAs is increasing as scientists are beginning to appreciate the broad role that these molecules play in the regulation of eukaryotic gene expression. There is speculation that miRNAs may represent a newly discovered layer of gene regulation. As a result, there is intense interest among researchers around the world in the targets and mechanism of action of miRNAs.

The two best understood miRNAs, lin-4 and let-7, regulate developmental timing in C. elegans by regulating the translation of a family of key mRNAs (Pasquinelli and Ruvkun G Ann Rev Cell Dev Biol. **18** (2002), 495-513). Several hundred miRNAs have been identified in C. elegans, Drosophila, mouse, and humans. As would be expected for molecules that regulate gene expression, miRNA levels have been shown to vary between tissues and developmental states.

In human, there are between 200 and 300 miRNA genes and about 200 have been identified at the moment. In heart, liver or brain, it is found that a single, tissue-specifically expressed miRNA dominates the population of expressed miRNAs and suggests a role for these miRNAs in tissue specification or cell lineage decisions (Lagos-Quintana et al. Current Biology **12** (2002), 735-739).

Characterization of a number of miRNAs indicates that they influence a variety of processes, including early development (Reinhart et al. Nature **403** (2000), 901-906), cell proliferation and cell death (Brennecke et al. Cell **113** (2003), 25-36), and apoptosis and fat metabolism (Xu et al. Curr. Biol. **13** (2003), 790-795). In addition, one study shows a strong correlation between reduced expression of two miRNAs and chronic lymphocytic leukemia, providing a possible link between miRNAs and cancer (Calin et al., Proc Natl Acad Sci USA **99** (2002), 15524-15529). Although the field is still young, there is speculation that miRNAs could be as important as transcription factors in regulating gene expression in higher eukaryotes.

miRNAs affects the expression of target genes by one of at least two mechanisms. Some bind to the 3'UTR of target mRNAs and suppress translation (Chi et al., Proc Natl Acad Sci USA. **100** (2003), 6343-6346). Others act as siRNAs, binding to and destroying target transcripts. miRNAs interfere with expression of messenger RNAs encoding factors that control developmental timing, stem cell maintenance, and other developmental and physiological processes in plants and animals. miRNAs are negative regulators that function as specific determinants, or guides, within complexes that inhibit protein synthesis (animals) or promote degradation (plants) of mRNA targets (Carrington and Ambros, Science. **301** (2003), 336-338). Plants with altered miRNA metabolism have pleiotropic developmental defects. In *Arabidopsis,* a miRNA has been identified "JAW" that can guide messenger RNA cleavage of several TCP genes controlling leaf development (Palatnik et al., Nature **425** (2003), 257-263).

Recently, miRNAs have been identified in undifferentiated and differentiated mouse embryonic stem (ES) cells (Houbaviy et al. Dev Cell **5** (2003), 351-358). Their expression is repressed as ES cells differentiate into embryoid bodies and is undetectable in adult mouse organs. In contrast, the levels of many previously described miRNAs remain constant or increase upon differentiation. These results suggest that miRNAs may have a role in the maintenance of a pluripotent cell state and in the regulation of early mammalian development.

Finally, miRNA mechanism of action is diverse and does not only target RNA transcript. miRNA's may also regulate gene expression by causing chromatin condensation. Several groups have shown that binding of dsRNAs to plant-promoter regions can cause gene silencing―an effect that is mediated via DNA methylation.

The detection of miRNA is difficult to perform given their diversity, their small size and their low copy number in the cells. Because their small size precludes the use of RT-PCR, most miRNA researches currently use Northern blot analysis with polyacrylamide gels and radioactive labeling to examine miRNA expression. This technique is labor intensive, requires large amounts of RNA and is restricted to the analysis of one miRNA at a time. Moreover, the use of radiolabeled probes is not recommended for routine tests because of their short half-life and the requirement of safe infrastructure and waste release.

Trancriptional regulation of multiple gene expression is a complex and subtle process. In order to investigate the effect of the miRNA on their transcribed genes, the assay has to be quantitative since small variation in their amount affects the gene expression in a significant way and modifies the cell composition.

Thus, there is a need in the art for a sensitive method to determine, whether a cell is subject to a RNAi mediated transcriptional regulation.

### Summary of the invention

It is, therefore, an object of the present invention to provide a method for rapidly and reliably detecting and quantifying the cellular transcriptional regulation mediated by RNAi due to the presence of naturally occuring miRNA.

In accomplishing these and other objects of the invention, there is provided, in accordance with one aspect of the invention, a method for determining the RNAi mediated transcriptional regulation of a cell by the determination of a pattern of at least 3 miRNA detected simultaneously and quantified in the same cell extract. The method of determination of a pattern of miRNA comprises the steps of: (i) providing an array onto which are fixed capture probes, said capture probes being arranged on pre-determined locations thereon and reflect the genomic or transcriptional matter of a cell, (ii) isolating miRNAs potentially present from a cell, (iii) elongating or ligating said miRNAs into target labeled polynucleotides, (iv) contacting said target labeled polynucleotides with the array under conditions allowing hybridization of the target labeled polynucleotides to complementary capture probes present on the array, (v) detecting and quantifying a signal present on a specific locations of the array, wherein the detection of a pattern of at least 3 signals on the array reflects the pattern of miRNA being involved in the RNAi mediated cellular transcriptional regulation.

The method according to the present invention may further comprise the step of correlating the cell transcriptional regulation provided by the detection and quantification of a pattern of miRNAs with the pattern of expression of the regulated genes in the same sample.

The presence and the amount of the different miRNA may be correlated with the amount of the different genes for which transcription is under the control of these different miRNA.

In one embodiment, the detected miRNAs are mature miRNAs. In one embodiment, the cellular transcriptional regulation provided by the detection and quantification of a pattern of is correlated with the pattern of expression of the miRNA targeted genes in the same sample. In another embodiment, the cell transcriptional regulation provided by the detection and quantification of a pattern of miRNAs is correlated with the pattern of expression of the genes having mRNA sequences complementary to the corresponding miRNA sequences detected in the same sample.

In another embodiment, the invention provides a method, wherein the cellular transcriptional regulation is related to one the following fields: development, cell differentiation or stem cell maintenance, cell proliferation, cell death, chromatin condensation or cell transformation.

In still another embodiment, the detection of the miRNA is performed after elongation of the miRNA hybridized on its complementary bait sequence with the Tth DNA polymerase 3. In another embodiment, the AMV or the M-MLV reverse transcriptase may be used for carrying out the elongation reaction.

In an alternative embodiment, the DNA/DNA-RNA hybrid complex obtained by elongation is then amplified by any linear amplification methods such as in vitro RNA transcription, asymetric or linear PCR. In a preferred embodiment, one primer is provided for linear amplification of the elongated sequences. Quantification of the multiple miRNA present in a sample is provided by one simple treatment of all the miRNA and direct hybridization on their corresponding capture

In another embodiment, the detection of the miRNA is performed after ligation of the miRNA hybridized on its complementary bait sequence with an adjacent probe. In another embodiment, the adjacent probe is pre-hybridized with its complementary bait sequence before ligation with the miRNA. In still another embodiment, the T4 RNA ligase may be used for carrying out the ligation reaction. In a preferred embodiment, the adjacent probe is labeled.

The invention further provides kits for the determination of cellular transcriptional regulation in a sample comprising an array comprising capture probes being arranged on pre-determined locations and having sequences identical or complementary to miRNAs of interest or parts thereof and optionally, buffers and labels. In another embodiment, the kit may also comprise a second array for the detection and quantification of the expression of the regulated genes in the same sample.

Other objects, features and advantages of the present invention will become apparent from the following detailed description. The detailed description and specific example, while indicating preferred embodiments, are given for illustrative purposes only, since various changes and modifications within the spirit and scope of the invention will become apparent to those skilled in the art from this detailed description. Further, the example demonstrates the principle of the invention and cannot be expected to specifically illustrate the application of this invention to all the examples where it will be obviously useful to those skilled in the prior art.

### Description of the drawings

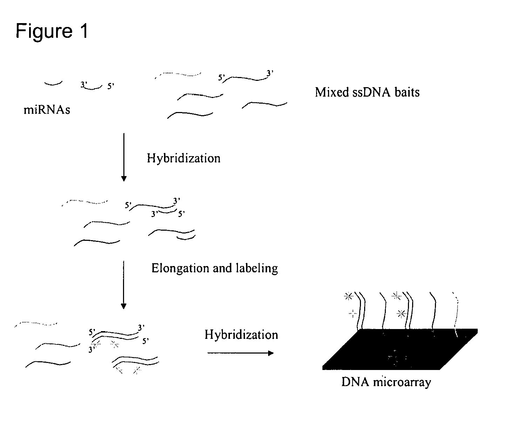

Figure 1 shows an embodiment for the detection of miRNAs on arrays. The miRNAs are first incubated in solution with a mixture of single strand DNA baits having part of their sequence complementary to the miRNAs. After elongation and labeling, they are detected by hybridization on array bearing sequences identical at least partly to the miRNA for which the analysis is required.

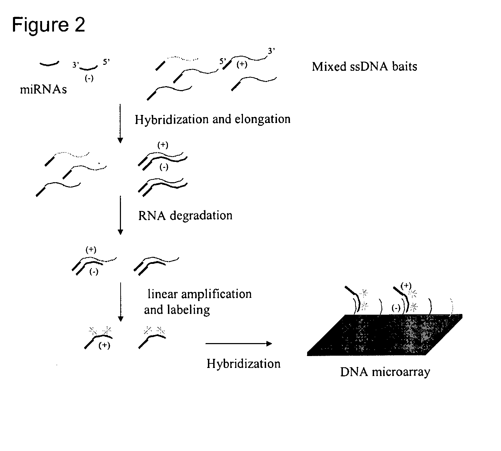

Figure 2 shows an alternative embodiment of figure 1. The single strand DNA baits are composed of three parts : the 3' end is complementary of the miRNA, the middle part is specific of each bait and the 5' end sequence is common to all baits. After hybridization of the miRNA and their elongation, the miRNA is degraded. A primer complementary of the common sequence of the elongated DNA is provided for linear amplification. Labeling occurs during the amplification by the incorporation of labeled nucleotides by a DNA polymerase. After linear amplification, the labeled products are detected on an array bearing sequences complementary at least partly to the amplified product.

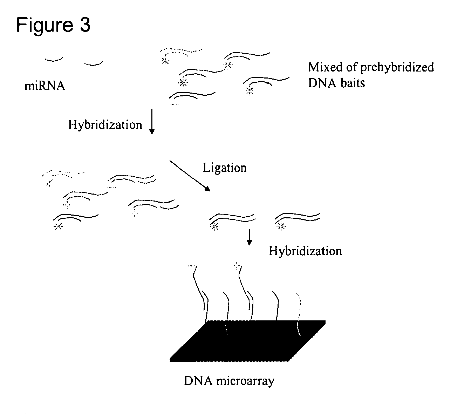

Figure 3 shows an embodiment for the miRNAs detection in which the miRNAs are hybridized with a mixture of single strand DNA baits which are pre-hybridized with labeled probes being adjacent to the miRNAs possibly present in the sample. After ligation of the miRNAs with the adjacent probes, the ligated products are detected on an array bearing sequences identical at least partly to the miRNA for which analysis is required.

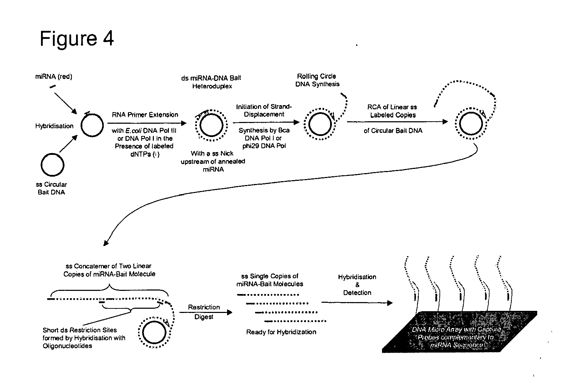

Figure 4 shows an embodiment for detection of miRNA by linear amplification of baits using rolling circle amplification. A pool of single stranded circular baits targeting one or more miRNA are hybridized in solution to the miRNA sample preparation. The annealed miRNAs then act as RNA primers for selected DNA-dependent DNA polymerases to initiate DNA synthesis on the miRNA-primed bait template molecule. The polymerase elongation is performed in the presence of labelled nucleotides. The RNA-primed bait -DNA polymerase reaction is further subjected to a second DNA polymerase with strong strand displacement activity to transform the initial primer extension reaction into a rolling circle amplification synthesis. The long single-stranded DNA molecules comprising DNA concatemers of miRNA sequences is fragmented to miDNA monomers to facilitate hybridisation with capture probes in the array. The fragmentation is achieved in a sequence-specific manner by hybridization to DNA-oligonucleotides having a length between 6 and 15 and preferably between 9 and 12,, which are complementary to a unique restriction endonuclease site placed downstream of the miRNA sequence on the bait DNA, followed by incubation with the corresponding restriction endonuclease. The miRNA specific polynucleotides are detected on a microarray presenting capture probes complementary to the amplified product.

### Detailed description of the invention

### Definitions

The term "genes" shall designate the genomic DNA which is transcribed into RNA and then optionally translated into peptides or proteins. The measurement of the expressed genes is performed on either molecules within this process most currently the detection of the mRNA or of the peptide or protein. The detection can also be based on specific property of the protein being for example its enzymatic activity.

The terms "nucleic acid, array, probe, target nucleic acid, bind substantially, hybridizing specifically to, background, quantifying" are as described in the international patent application WO97/27317, which is incorporated herein by reference.

The term "nucleotide triphosphate" refers to nucleotides present in either as DNA or RNA and thus includes nucleotides which incorporate adenine, cytosine, guanine, thymine and uracil as bases, the sugar moieties being deoxyribose or ribose. Other modified bases capable of base pairing with one of the conventional bases adenine, cytosine, guanine, thymine and uracil may be employed. Such modified bases include for example 8-azaguanine and hypoxanthine.

The term "nucleotide" as used herein refers to nucleotides present in nucleic acids (either DNA or RNA) compared with the bases of said nucleic acid, and includes nucleotides comprising usual or modified bases as above described. References to nucleotide(s), oligonucleotide(s), polynucleotide(s) and the like include analogous species wherein the sugar-phosphate backbone is modified and/or replaced, provided that its hybridization properties are not destroyed. By way of example, the backbone may be replaced by an equivalent synthetic peptide, called Peptide Nucleic Acid (PNA).

The terms "nucleotide species" is a composition of related nucleotides for the detection of a given sequence by base pairing hybridization; nucleotides are synthesized either chemically or enzymatically but the synthesis is not always perfect and the main sequence is contaminated by other related sequences like shorter one or sequences differing by a one or a few nucleotides. The essential characteristic of one nucleotides species for the invention being that the overall species can be used for capture of a given sequence.

"Polynucleotide" sequences that are complementary to one or more of the miRNA described herein, refer to capture probes that are capable of hybridizing under stringent conditions to at least part of the nucleotide sequence of said miRNA or miRNA copies. Given the small size of the miRNA, the capture molecules have to be identical or at least have more than 90% identical sequence in order to specifically detect the miRNA beside other possible flanking regions such as spacer sequences.

"Bind(s) substantially" refers to complementary hybridization between a probe nucleic acid and a target nucleic acid and embraces minor mismatches that can be accommodated by reducing the stringency of the hybridization media to achieve the desired detection of the target polynucleotide sequence.

The terms "capture probe" designates a molecule which is able to specifically bind to a given polynucleotide. Polynucleotide binding is obtained through base pairing between two polynucleotides, one being the immobilized capture probe and the other one the target to be detected.

The term, the genomic or transcriptional matter of a cell shall designate the genes and/or the genome and/or the transcripts represented by RNA in the cell.

The term miRNA is a non coding small RNA produced by a DICR enzyme from a double stranded RNA Precursor. The precursor has a stem loop or hair-pin structure. miRNA are present in animals or plants. They are mainly bound to a prtein complex termed miRISCs. They represent one of the component of the RNAi beside other ones like the siRNA.

The present invention is based on the use of arrays having multiple single nucleotide sequences arranged on specific, pre-determined locations thereon and being identical or complementary to miRNA, present in the cells for which the pattern of transcriptional regulation is to be determined.

The main characteristic of the invention is to obtain a pattern of transcriptional regulation based on the simultaneous detection and quantification of multiple miRNAs present in a cell. The signals of the different spots related to each gene being a direct measurement of the diversity and the concentration of the miRNA in the analysed cells or tissues. Also, the invention is not limited by the number of miRNA to be screened. The array allows to analyse either from 5 to 500 and more preferably until 5000 miRNAs in a cell. This number depends on the species and the number of expressed miRNA genes in the analysed cells.

The present invention provides a method for the determination of cellular transcriptional regulation by the simultaneous detection and quantification of multiple miRNAs present in a cell on an array and by detecting a signal present on a specific location on the array, said signal at such location being related to the presence of one miRNA with the detection of at least 3, preferably at least 5 , more preferably at least 10 and even more preferably at least 20 miRNAs on the array being indicative of a given miRNA or RNAi mediated cellular transcriptional regulation.

In general, in a cell, there are typically about 20 miRNA genes expressed. In human there are about 200 to 300 miRNA genes. The identification of a pattern of expressed miRNAs in a given cell brings an answer to the question, whether a cell is subject to RNAi mediated transcriptional regulation (e.g. the genes regulated by these miRNA and their target genes). In a preferred embodiment, to unravel the cellular transcriptional regulation, the pattern of at least 3 miRNAs obtained by the method of the invention is correlated with the pattern of expression of the regulated genes in the same sample (e.g provided by a second array). In another embodiment, the pattern of at least 3 miRNA is correlated with the pattern of expression of the miRNA target genes in the same sample (e.g provided by a second array). In an alternative embodiment, the pattern of at least 3 miRNA is correlated with the pattern of expression of genes having mRNA sequences complementary to the corresponding miRNA sequence in the same sample (e.g. provided by a second array). In another embodiment, the pattern of at least 3 miRNAs obtained by the method of the invention is correlated with activated transcriptional factors in the same sample.

In a preferred embodiment, the invention provides a method for the simultaneous detection of at least 3 of the miRNA presented in Table 1 for human cells and at least 3 miRNA presented in Table 2 for mouse cells.

In a preferred embodiment, the invention provides a method for the simultaneous detection of at least 3 of the miRNA presented in Table 3 for human cells. Each individually detected miRNA from Table 3 regulates one or several genes. A list of miRNA sequences and their targeted genes are available in John B, Enright AJ, Aravin A, Tuschl T, Sander C, et al. (2004) Human microRNA targets. PLoS Biol 2(11) : e363. and on www.microrna.org. The present invention covers the detection of part or all of the miRNA presented in this publication and data bank.

In a preferred embodiment, the invention provides a method wherein the cellular transcriptional regulation is related to one the following fields: development, cell differentiation or stem cell maintenance, cell proliferation, cell death, chromatin condensation or cell transformation.

In principle, the method comprises the steps (step (i)) of providing a support containing an array onto which a number of capture probes are arranged on pre-determined locations. These capture probes relate to miRNA sequences or their complement and/or genes and/or transcripts of a cell.

The support is generically composed of a solid surface which may be selected from the group consisting of glasses, electronic devices, silicon supports, silica, metal or mixtures thereof prepared in format selected from the group of slides, discs, gel layers and/or beads. Beads are considered as arrays in the context of the present invention, as long as they have characteristics which allow a differentiation from each other, so that identification of the beads is correlated with the identification of a given capture probe and so of the target sequence

On the support, a number of capture molecules is fixed by covalent binding, each capture molecule being located at a specific location and having at least in part a sequence in a single strand form identical to a miRNA or its complement for which the presence is screened.

In principle, the detection of the miRNAs may be performed on the same strand of the corresponding miRNA sequence (-) or on its complementary sequence (+), since the miRNAs contain one strand (-). However, in some applications, the miRNAs may be complementary to mRNA (+) and the use for the detection of strand identical to the miRNA sequences (-) might lead to a binding of the natural mRNA (+) as well, which might interfere with the analysis. This is the case for the detection method presented in figure 1 and 3. In this case, the sequence (+) complementary to the miRNA (-) has to be present on the surface of the array. In the method proposed in figure 2, there is not interference with mRNA since the detected sequences have the identical strand.

Generally, the capture probes may be synthesized by a variety of different techniques, but are preferably synthetized by PCR amplification from cloned genes using an aminated primer. The amino group of the amplicon is then reacted with a functionalized surface bearing reactive groups, such as, but not limited, to aldehyde, epoxide, acrylate, thiocyanate, N-hydroxysuccinimide. After having formed a covalent linkage, the second strand of the amplicon is then removed by heating or by alkaline treatment so that single strand DNA or RNA is present on the surface and ready to bind to the miRNA or its complement.

Given the progress of chemical synthesis of the nucleotides, the use of chemically synthetized nucleotides is also envisaged in the invention. The synthetized nucleotides are also preferably aminated or thiolated and deposited on the functionalized surface. Advantage of the chemically synthetized nucleotides is their easy production but the limitation is the yield of each of the successive addition of nucleotides making the capture probes not unique especially when long polynucleotide capture probes are synthetized. We will then considered a chemically synthetized capture probe typically as a capture probe species.

In a preferred embodiment the array comprises capture probes represented by polynucleotides having a sequence identical to miRNA. In another embodiment, the array contains capture probes being polynucleotides having a sequence complementary to miRNA.

Preferably, the different capture molecules present on the array cover most and preferably all of the miRNA present in a cell. In another prefered embodiment, the capture molecules allows the binding of the miRNA related to the regulation of the gene of interest in the given application. In an embodiment, the array comprises 5-200, preferably 5-500, 5-1000 and even 5-5000 capture probes specific of the miRNA or their complement.

In principle, the micro-array contains at least 3 capture probes, e.g. one capture probe associated with a miRNA. Yet, the number of capture probes on the micro-array may be selected according to the need of the skilled person and may contain capture probes for the detection of up to about 5000 different miRNA, e.g. about 100, or 200 or 500 or 1000, or 2000 different miRNA involved in the cell transcriptional regulation.

In a preferred embodiment, the array may target human or mouse miRNA sequences as listed in Table 1 and Table 2 respectively. Similar array can be constructed for other species including rat, Drosophila, Arabidopsis and Caenorhabditis with some adaptation in the sequence for the capture probes.

In another embodiment, the array may also target human miRNA sequences as listed in Table 3. This Table presents some miRNA and some of their target genes.

According to a preferred embodiment the capture probes are polynucleotides and are unique for each miRNA to be detected and quantified on the array. The minimum length corresponds to the size of the miRNA or their complement. The capture probes may comprise a spacer sequence which is not related to the miRNA sequences and which do not react significantly with these sequences. Spacer to be used in the present invention may be obtained following methods described in the WO0177372 which document is incorporated herein by way of reference. The overall length of the capture probes (including the spacer) may range from about 15 to about 1000 nucleotides, preferably of from about 15 to about 400, or 15 to 200 nucleotides, or more preferred of from about 15 to about 100, or from 15 to 50, or from 15 to 30 and are fixed on a support being any solid support as long as they are able to hybridize with their corresponding miRNA or their complement and be identified and quantified.

In a preferred embodiment, the capture probes have sequences which are identical for at least 10 to 1000 nucleotides to the same part of the mRNA corresponding to the miRNA to be detected. In another embodiment, an individual capture probe is used for the detection of several miRNAs directed to the same mRNA.

In a preferred embodiment, the capture probes are chosen in order to obtain a quantitative hybridization of the target nucleotides meaning a reproducible detection with a coefficient of variation below 30% and preferably below 20% for the same target nucleotide in separated experiments and below 50% and preferably below 25% for different target nucleotides present in the same solution.

In another preferred embodiment, the capture molecules are present at a density superior to 10 fmoles, and preferably 100 fmoles per cm² surface of the solid support. In another embodiment capture probes are present on different supports being preferentially beads with chemical or physical characteristics for their identification with a specific capture probe.

The invention also embraces the support and its substrate on which is bound the capture molecules for the detection of given mRNA target molecules which are complementary to the corresponding miRNA sequences.

Methods of arranging nucleotides and polynucleotides on an array are well known in the art and may be found in Bowtell, D. and Sambrook (DNA Microarays, J. Cold Spring Harbor Laboratory Press, 2003 Cold Spring Harbor, New York, 1-712) which document is incorporated herein by way of reference. In a preferred embodiment the nucleotide sequence is attached to the support via a linker, which may be a polynucleotide exhibiting a length of between about 20 to 200 nucleotides (WO0177372) . In principle, the capture probes may be DNA, PNA or RNA.

In a next step (step (ii)), miRNA from a cell of interest is isolated. An exemplary process for the isolation of small interference RNA (siRNA) is described e.g. in Tuschl et al. (Genes & Dev. 13 (1999), 3191-3197), which document is incorporated herein by way of reference.

The miRNA once isolated will be labeled prior to its use (step(iii)). The miRNA is incorporated into a labeled DNA-RNA sequence which is then detected on the array. The labeling may be performed by elongating or ligating the miRNA. In a preferred embodiment, the labeling may be performed by incubating the miRNA with a mixture of ssDNA under conditions as to obtain formation of a RNA-DNA hybrid complex, whereupon an elongation and concurrent labeling of the small miRNA may be achieved. Here, the ssDNA bait is used as a matrix and labeled ribonucleotides/deoxynucleotides are utilized for the elongation of miRNA..

The ssDNA bait used for the formation of the hybrid complex can be replaced by any nucleotide or nucleotide-like molecules as long as the elongation of the bound miRNA is possible. After denaturation, the labeled strand will be used for incubation with the capture probes present on the array for detection and quantification of the miRNA (figure 1).

According to a preferred embodiment, the elongation is performed with the Tth DNA polymerase 3 which accept as primer RNA sequences such as miRNAs. The elongated and labeled polynucleotide is DNA. In another embodiment, the elongation is obtained with the AMV or with the M-MLV reverse transcriptase.

In a preferred embodiment, the ssDNA bait is a sequence identical to the mRNA strand or part of it (+) which is complementary to the corresponding miRNA (-) for which the analysis is required. After elongation and labeling, the elongated strand (-) is hybridized on a capture probe (+) identical to the mRNA strand or part of it. The same capture probe may be used for parallel detection and quantification of the mRNA present in the same sample. After retro-transcription of the mRNA (+), the labeled cDNA (-) is hybridized on the same capture probe. This method greatly simplifies the production of the capture probes which are equivalent for both applications.

In another embodiment, detection of the miRNA and their precursor is accomplished by providing a specific synthetic bait DNA polynucleotide during a labelling reaction using the complete DNA polymerase I, E.coli DNA polymerase III or Tth DNA polymerase III holoenzyme. The RNA nucleotides complementary to the DNA baits serve as primers for the DNA polymerase extension. The bait is designed to bind either the one of the miRNA strands or to a nucleotide sequence exclusively present in the precursor forms of miRNA.

This bait contains further a nucleotide extension allowing for incorporation of multiple labelled nucleotides and contain in its 3' end a series of nucleotides that serve as complements to the microarray capture probe. The labelled nucleotide incorporation is maximised by using an optimized sequence composition allowing for multiple labelled nucleotides to be incorporated with high efficiency. The 3' end of the bait is designed with a sequence tag that is unique for each RNA molecule and hybridize to a complementary capture probe of the microarray. In this case, the array is a standard array of barcode tagged capture probes, and the specificity is provided by the bait in the labelling step. Baits are designed with a nucleotide sequence specific for each detection application. The same enhancement strategy using LNA can be used.

In a preferred embodiment, the mixture of ssDNA baits is composed of three parts : the 3' end is complementary of the miRNA, the middle part is specific of each bait and the 5' end sequence is common to all baits. After hybridization of the miRNA and their elongation, the product is amplified. After degradation of the miRNA, the matrix for the amplification is a DNA/DNA hybrid complex. A primer complementary of the common sequence of the elongated DNA is provided for linear amplification with a DNA polymerase. Advantageously, only one primer is required for the amplification of all elongated miRNAs. Altered cycles of denaturation, primer annealing and polymerisation are performed like in a normal PCR except that only one primer is used which results in a linear amplification. The advantage of such amplification is that quantification of the initial amount of miRNA remains possible due to the linearity of the amplification. After linear amplification, the products are detected on an array bearing sequences complementary at least partly to the amplified product (figure 2). The target labeled nucleotides which are hybridized on the array are preferably labeled during the amplification. Preferably, the capture probes of the array do not comprise the primer sequence used for the amplification nor the miRNA sequences or their complement. In order to avoid interference between the ssDNA baits (+) introduced at the beginning of the assay with the amplified labeled products (+) for the hybridization on the capture probes (-) of the array, the mixture of ss DNA baits may be specifically degraded before the amplification (e.g. by S 1 nuclease).

Alternatively, the primer complementary of the common sequence of the elongated DNA may comprise a T7 promoter sequence for an RNA polymerase that might be used for in vitro transcription. The primer may also comprise a Tag sequence which is used for further amplification (e.g. the tag may be a sequence rich in cytosine if the amplification is performed with labeled CTP, thus increasing the number of incorporated label during the amplification).

The labeling is preferably obtained by the incorporation of labeled ribonucleotides/deoxynucleotides during the amplification step in order to obtain target labeled polynucleotides according to the invention. Fluorescent labeled nucleotides are preferred since they are incorporated by the polymerase and lead to the formation of fluorescent labeled target polynucleotides. Cy3, Cy5 or Cy7 labeled nucleotides are preferred fluorochromes.

In another embodiment, the detection of the miRNA is performed after ligation of the miRNA hybridized on its complementary bait sequence with an adjacent probe.

The labeling may be performed by ligating the miRNA. In a preferred embodiment, the labeling of the miRNA is performed after ligation of the miRNA hybridized on its complementary bait sequence with an adjacent probe. Labeling may be obtained by using a labeled adjacent probe for ligation. In a preferred embodiment, the adjacent probe is pre-hybridized with its complementary bait sequence before ligation with the miRNA. Ligation may be performed under conditions as to obtain formation of a DNA/DNA-RNA hybrid complex. Here, the DNA bait is used as a matrix and the DNA adjacent probe is utilized for ligation with miRNA. In a preferred embodiment, the ssDNA bait is a sequence identical to the mRNA strand or part of it which is complementary to the corresponding miRNA for which the analysis is required. The DNA baits used for the formation of the hybrid complex can be replaced by any nucleotide or nucleotide-like molecules as long as the ligation of the bound miRNA is possible. After denaturation, the labeled strand will be used for incubation with the capture probes present on the array for detection and quantification of the miRNA (figure 3). Preferably, the capture probes of the array are not complementary of the labeled adjacent probes in order to avoid false positive hybridizations.

According to a preferred embodiment, the ligation is performed with the T4 RNA ligase which accept to ligate DNA sequences to RNA sequences such as miRNAs.

Preferred labels may be detected, e.g. via fluorescence, colorimetry, chemo- or bioluminescence, electric, magnetic or particularly biotin. Biotin-labeled nucleotides may be attached/incorporated, which is then recognized by binding proteins being either antibodies or streptavidin or related binding molecules. The binding proteins are labeled by any chemical or physical means and detected and quantified. Indirect labeling is also of use when amplification of the signal is required.

Detection of miRNA can be further enhanced by using a polynucleotide amplification step. This is accomplished using a mixture of DNA polymerase III or I of E. coli with a strand displacement DNA polymerase (ex. Bca DNApol I or phi29 DNApol) and circular DNA polynucleotide baits that are complementary to the sequence (miRNA or precursor) to be targeted. When the baits are annealing to their target sequences, a single strand concatenated polynucleotide is synthesized by the DNA polymerase. Labelled nucleotides are provided for incorporation during this amplification step. The resulting labelled single strand concatenated molecule is then hybridized on the microarray presenting complementary capture probes. The Single stranded DNA concatemer can be fragmented by hybridizing short oligonucleotides that reconstitute restriction sites. As an option, the concatenated molecule is fragmented by for ex. mild DNase treatment.

### a) Preparation of the circular baits:

Two methods are prefered to prepare circular bait molecules in large scale.
1. They are produced by annealing the extremities of a linear single stranded bait DNA polynucleotide to a shorter (ex: 40 - 50 bases) single stranded polynucleotide. The overlapping sequence of both ends of the larger molecule is typically 25 bases and is complementary to the 50 bases polynucleotide. The annealed molecules are then treated by a DNA ligase specialised in ligation of single-stranded nicks in ds DNA molecules producing a circular bait polynucleotide. One preferred enzyme is the NAD⁺-dependent E.coli DNA ligase. The E. coli DNA ligase joins the 5'-end of the ss bait polynucleotide to its 3'-end when they are annealed next to each other on the shorter polynucleotide.
2. The ends of individual single-stranded bait molecules are ligated directly without preliminary hybridisation to a complementary short polynucleotide. This reaction is catalysed by a ssDNA Ligase specialised on intramolecular ligation of single-stranded DNA polynucleotides (CircLigase^{TM} ssDNA Ligase, Epicentre Technologies, CL4111K). In both cases, after the ligation the excess of the shorter (50 bases) molecule (annealed or free in solution) as well as non-ligated linear bait molecules are then removed with an exonuclease. A number exonuclease enzymes are preferably used for that purpose, comprising but not limited to exonuclease I, mung bean exonuclease, bacterial DNA polymerase III epsilon subunits or DNA polymerases with a strong 3'-5' proof-reading exonuclease activity. After the incubation the exonucleases are inactivated by a heat treatment at 90-95°C.

### b) miRNA annealing in solution and detection of the amplified product on microarray.

A pool of single stranded circular baits (as prepared in step a) targeting one or more miRNA's and precursor RNA molecules are hybridized in solution to the miRNA sample preparation. The annealed miRNAs then act as RNA primers for selected DNA-dependent DNA polymerases, preferably but not limited to the alpha subunits of bacterial DNA polymerases III or E.coli DNA polymerase I, to initiate DNA synthesis on the miRNA-primed bait template molecule. The RNA-primed bait -DNA polymerase reaction is further subjected to a second DNA polymerase with strong strand displacement activity, such as Bca DNA Pol I, Bst DNA Pol I or phi29 DNA polymerase, to transform the initial primer extension reaction into a rolling circle amplification synthesis (RCA). After rolling circle amplification and labelling, the polynucleotides are detected on a microarray presenting capture probes complementary to the amplified product (natural sequences or tags included in the bait). Optionally, the long single-stranded DNA molecules comprising DNA concatemers of miRNA sequences can be fragmented to miDNA monomers to facilitate hybridisation with capture probes in the array. The fragmentation is achieved in a sequence-specific manner by hybridisation to nonamer DNA-oligonucleotides, which are complementary to a unique restriction endonuclease site placed downstream of the miRNA sequence on the bait DNA, followed by incubation with the corresponding restriction endonuclease.

### c) miRNA annealing in solid phase and on site accumulation of the amplification products on the microarray without hybridization step.

The single stranded circular baits are immobilized on discrete regions at the surface of a substrate compatible with rolling circle amplification. The synthesis products, preferably labeled, then accumulated on site (on spot). In this case the circular RCA template (bait DNA) is surface-immobilized, whereas the other reactants (e.g. DNA polymerases, labelled and non-labeled dNTPs) are free in solution that covers the array surface.

In a preferred embodiment, elongation of the miRNAs is effected on complementary bait sequences being circular and single stranded. In an embodiment, the elongated miRNAs are amplified by rolling circle.

In another preferred embodiment, the bait sequences being circular and single stranded are capture probes arranged on pre-determined locations of an array.

In a next step (step (iv)), the labeled miRNA or molecule derived therefrom (e.g. a DNA-copy or amplified RNA), is contacted with the array under conditions, allowing hybridization of the labeled miRNA, or the molecule derived therefrom, with the capture probes present on the array. After a time sufficient for forming the duplex, a signal is detected on a specific location on the array (step (v)). The detection of a pattern of at least 3 signals on the array reflects the pattern of miRNA being involved in the RNAi mediated cellular transcriptional regulation.

In a preferred embodiment, the signal present on a specific location on the array corresponds to a pattern of at least 5, 10, 15, 20, 25, 30 and even 50 miRNAs.

In a preferred embodiment, the signal associated with a cpature moleclue on the array is quantified. The preferred method is the scanning of the arrays with a scanner being preferentially a laser confocal scanner such as "ScanArray" (Packard, USA). The resolution of the image is comprised between 1 and 500 µm and preferably between 5 and 50 µm. To Preferably the arrays is scanned at different photomultiplier tube (PMT) settings in order to maximize the dynamic range and the data processed for quantification and corrections with the appropriated controls and standards (de Longueville et al, Biochem Pharmacol. **64 ,** 2002,137-49).

In case the miRNA, or molecule derived therefrom, has been labeled prior to the hybridization step, the presence of fixed labeled target will be indicative of the presence of miRNA in the sample. The amount of fixed labeled target on the array will be proportional to the miRNA if performed under the appropriate conditions.

The present invention is also particularly suitable to detect and/or quantify the processed miRNA but also their precursors preferably the Pre-miRNA. The detection of precusrsor miRNA transcripts is achieved by using for each miRNA particular capture probes on the microarray that will be complementaty to some parts of the Pre-miRNA but located outside the 20-25 nt bound to the RISC and having no effect on the transcription, preferably sequences present in the loop.

In a preferred embodiment, the capture probes of the array are able to detect both precursor and mature miRNA forms. Simultaneous detection of the precursor pool and the processed or mature form of miRNA in a cell allows a more detailed understanding of the regulatory state of the cell for transcription.

In an embodiment, the capture probes contains LNA (locked nucleic acid) nucleotides. The detection of miRNA can be enhanced by using a capture probe with LNA nucleotide in the positions of the mismatches of the miRNA duplex.

The presence of target bound on the different capture probes present on the solid support may be analyzed, identified and quantified by an apparatus comprising a detection and quantification device of a signal formed at the location of the binding between the target molecule and the capture molecule, preferably also a reading device of information recorded on a surface of said solid support, a computer program for recognizing the discrete regions bearing the bound target molecules upon its corresponding capture molecules and their locations, preferably also a quantification program of the signal present at the locations and a program for correlating the presence of the signal at these locations with the diagnostic and the quantification of the components to be detected according to the invention.

Therefore, the method as described herein may be utilized as part of a diagnostic and quantification kit which comprises means and media for performing an analysis of biological samples containing target molecules being detected after their binding to the capture probes being present on the support in the form of array with a density of at least 5 different capture probes per cm² of surface of rigid support.

Also provided by the present invention is a kit for the determination of cell transcriptional regulation in a sample, which kit comprises an array, harboring capture probes having a sequence identical or complementary to a miRNA or parts thereof and being present at pre-determined locations of the array, and buffers and labels. In another embodiment, the kit may also comprises a second array for the detection and quantification of the expression of the regulated genes in the same sample. The aim of this second array is to obtain a direct analysis and an overview of the genes which are essentially affected by the regulation through miRNAs present in the cells. In another embodiment, the detection and quantification of the expression of the regulated genes is obtained through the detection and quantification of mRNA or proteins in the same sample. In a preferred embodiment, the two arrays are present on the same support and allow a direct comparison between the pattern of miRNAs present in the sample with the pattern of genes differentially expressed in the same sample. In another embodiment, the two arrays are present on different supports. In another embodiment, the capture probes of the arrays for the detection of the miRNAs and of the mRNA are nucleotide sequences having part of their sequence identical to the mRNA. The advantage of such system is that the same capture probes present on the same support may be used for the detection of both the miRNA and their corresponding mRNA.

The signals of the different spots related to each gene are a direct measurement of the diversity and the concentration of the expressed genes (mRNA) in the analysed cells or tissues. The amount of genes is directly related to the presence and amount of the corresponding miRNA and their regulating activity of the corresponding genes. In this embodiment, the results obtained on the gene expression array is directly related to the results obtained on the miRNA array and conclusions may be drawn on the transcriptional regulation of these individual genes in the particular analysed sample. In particular, the variation in the amount of one miRNA in a test sample compared to a reference sample may be correlated with the change in the amount of transcribed mRNA corresponding gene. This correlation may be performed for at least 3 miRNA and a pattern of transcriptional regulation provided by the presence of the miRNA will be constructed. Such regulation may than be correlated to the biological situation from which the sample was derived for the analysis, being a biological or pathological or an experimental set up.

The invention is not limited by the number of genes to be screened. The array allows to analyse either from 2 to 100 and more preferably until 1000 and still up the entire gene pool present in a cell. This number depends on the species but can be as large as around 40.000 for the human genome.

The following example illustrates the invention without limiting it thereto.

### Example

The experiment is performed as schematically described in figure 1.

### miRNA extraction:

miRNAs are extracted from the a fresh (or frozen) pellet of 10² to 10⁷ cultured cell or tissues using the mirVana miRNA isolation procedure variant for isolation of RNA that is highly enriched for small RNAs (Ambion). The sample was disrupted in a denaturing lysis buffer and subsequently extracted in Acid-Phenol:Chloroform (Chomczynski and Sacchi, Anal. Biochem. **162** (1987), 156-159) 1/3 volume of 100% ethanol is added to the aqueous phase recovered from the organic extraction, mixed and passed through a glass filter cartridge (using centrifugal force). After this step, the filtrate was further enriched by adding 2/3 volume of 100% ethanol, mixed and applied on a second glass filter cartridge. The small RNA molecules remain trapped on the glass filter and are washed three times with a 45% ethanol solution. The RNA is then eluted with nuclease-free water and recovered in a collection tube.

### miRNA labelling:

The small size RNA population is then annealed, with a mixture of single stranded DNA molecules by heating at about 95°C for 5min and annealing for 30 min at 37°C. The annealed RNA-DNA hybrids are then labeled with biotin labeled dNTPs using Tth DNA polymerase 3 holoenzyme. miRNA strand extension is accomplished by incubation of the RNA-DNA mixture with Tth DNA polymerase 3 holoenzyme mix for 20 s. in a buffer containing 20mM Taps-Tris pH 7.5, 8mM Mg(OAc)2, 0.04 mg/ml BSA, 40 µM Sorbitol, 0.5mM ATP and 0.2mM labeled dNTP mix). The reaction is quenched by adding EDTA at a final concentration of 10 mM, vortexing and is maintained on ice.

### Hybridization:

The resulting product is then hybridized on the DualChips miRNA micro-array bearing ssDNA capture probes specific for miRNA sequences (Eppendorf, Hamburg, Germany).

Hybridization chambers were from Biozym (Landgraaf, The Netherlands). Hybridization mixture consisted in biotinylated miRNA-DNA hybrid, 6.5 µl HybriBuffer A (Eppendorf, Hambourg, Germany), 26 µl HybriBuffer B (Eppendorf, Hambourg, Germany), 8 µl H2O, and 2 µl of positive hybridization control.

Hybridization was carried out overnight at 60°C. The micro-arrays were then washed 4 times for 2 min with washing buffer (B10.1X + Tween 0.1 %) (Eppendorf, Hamburg, Germany).

The micro-arrays were than incubated for 45 min at room temperature with the Cy3-conjugated IgG Anti biotin (Jackson Immuno Research laboratories, Inc #200-162-096) diluted 1/1000 X Conjugate-Cy3 in the blocking reagent and protect from light.

The micro-arrays were washed again 5 times for 2 min with washing buffer (B1 0.1X + Tween 0.1 %) and 2 times for 2 min with distilled water before being dried under a flux of N2.

After image acquisition, the scanned 16-bit images are imported to the software, 'ImaGene4.0' (BioDiscovery, Los Angeles, CA, USA), which is used to quantify the signal intensities. The spots intensities are first corrected by a subtraction of the local background intensity from signal intensity.

In order to evaluate the entire experiment, several positive and negative controls (for hybridization and detection) are first analysed. Then the signal obtained on each miRNA spots is analysed in order to correlate the result with the presence or not of miRNA directed against the specific gene in the sample.

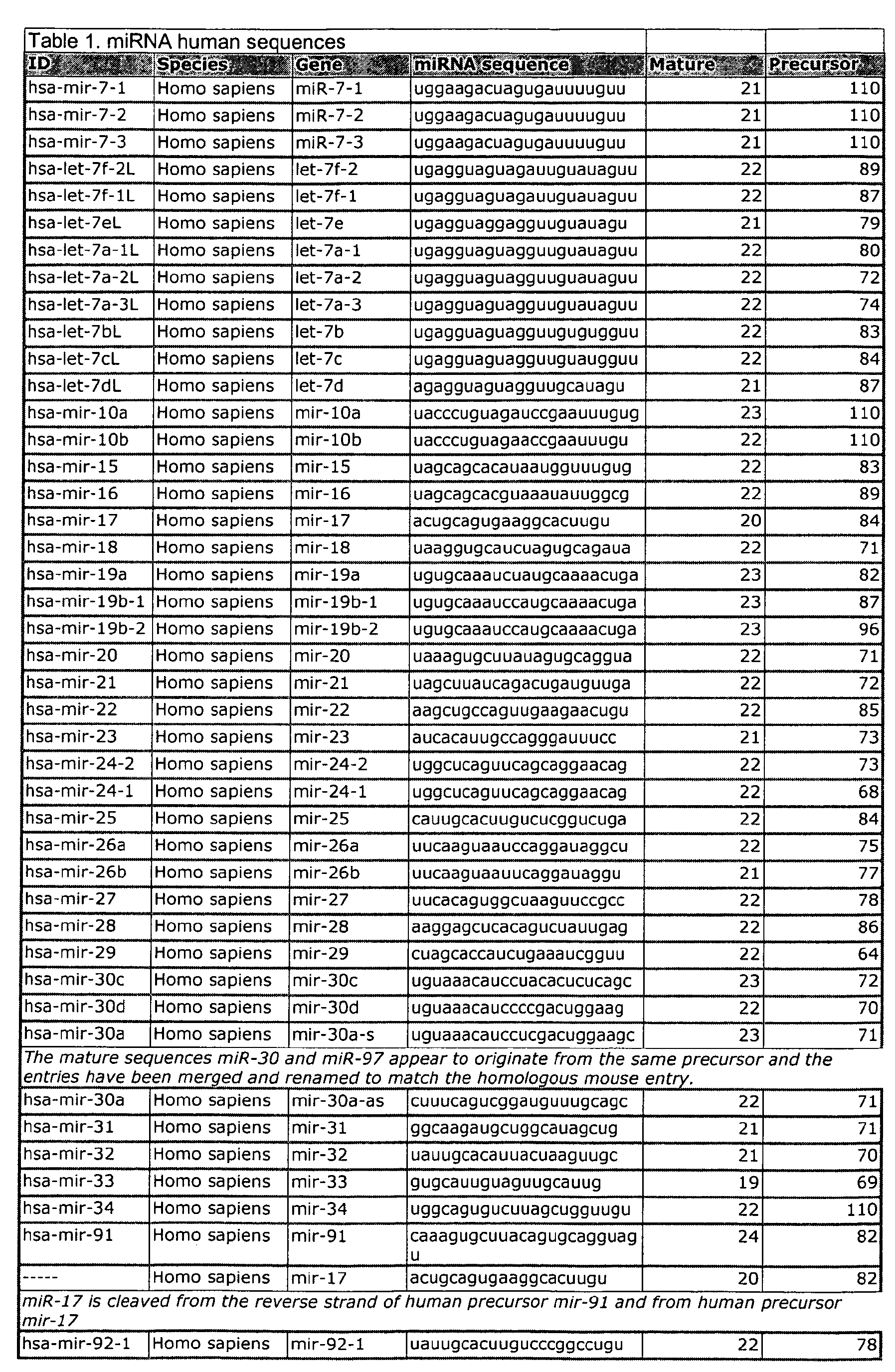

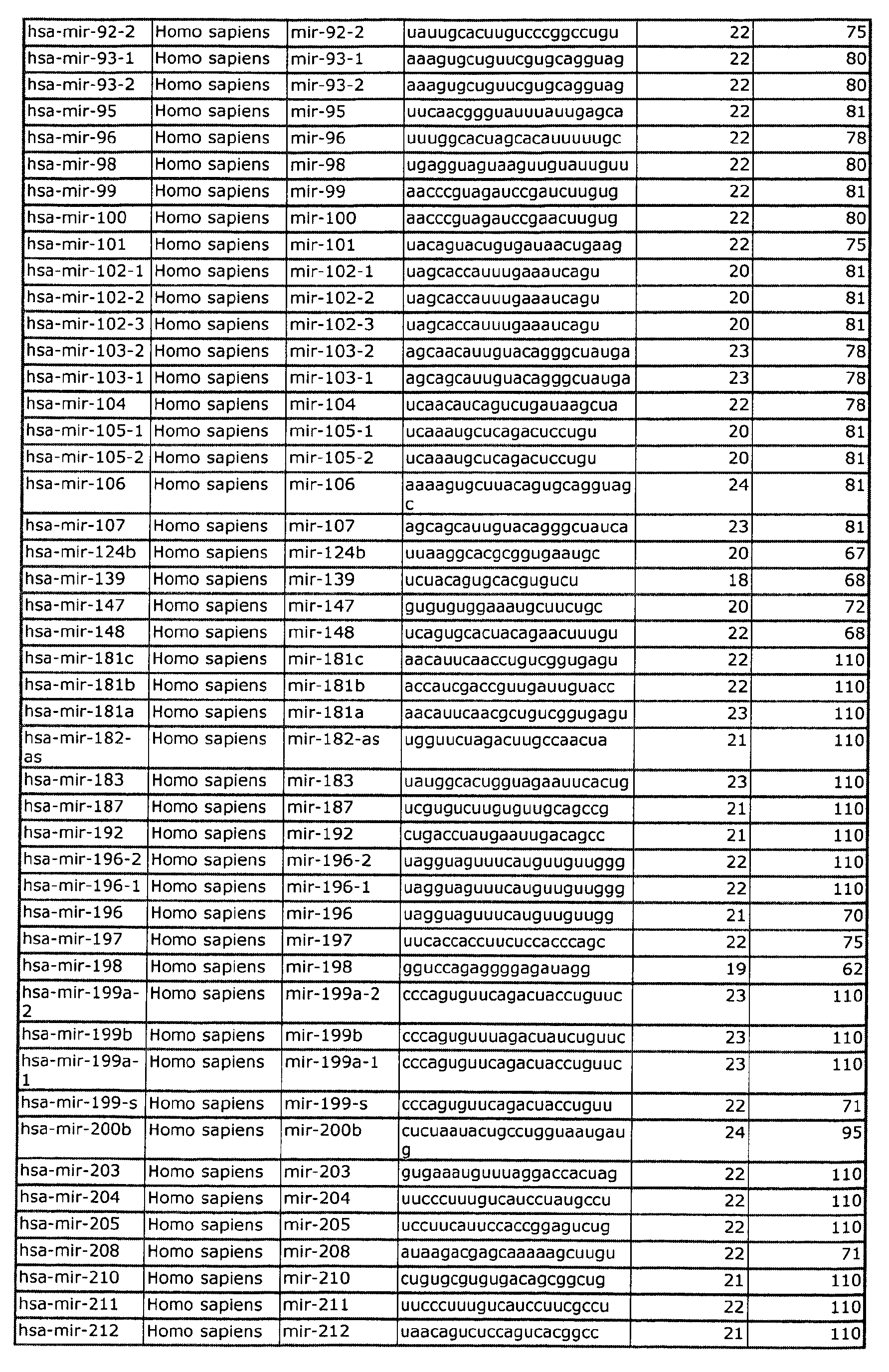

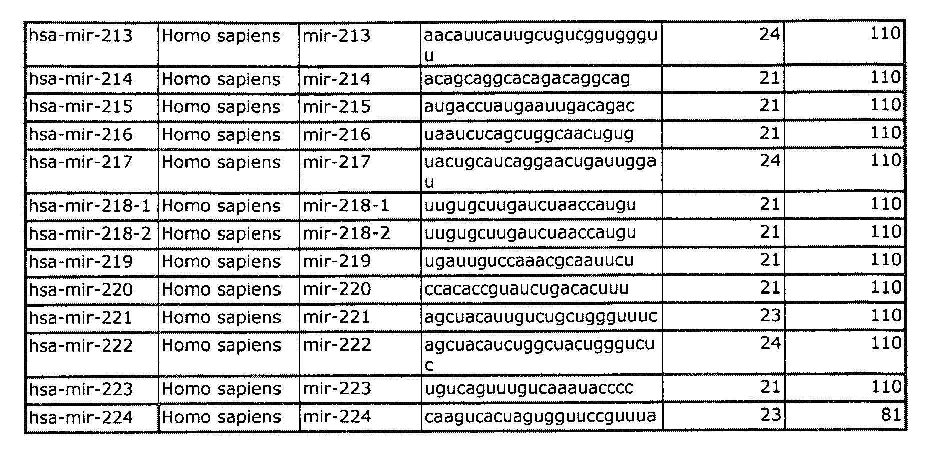

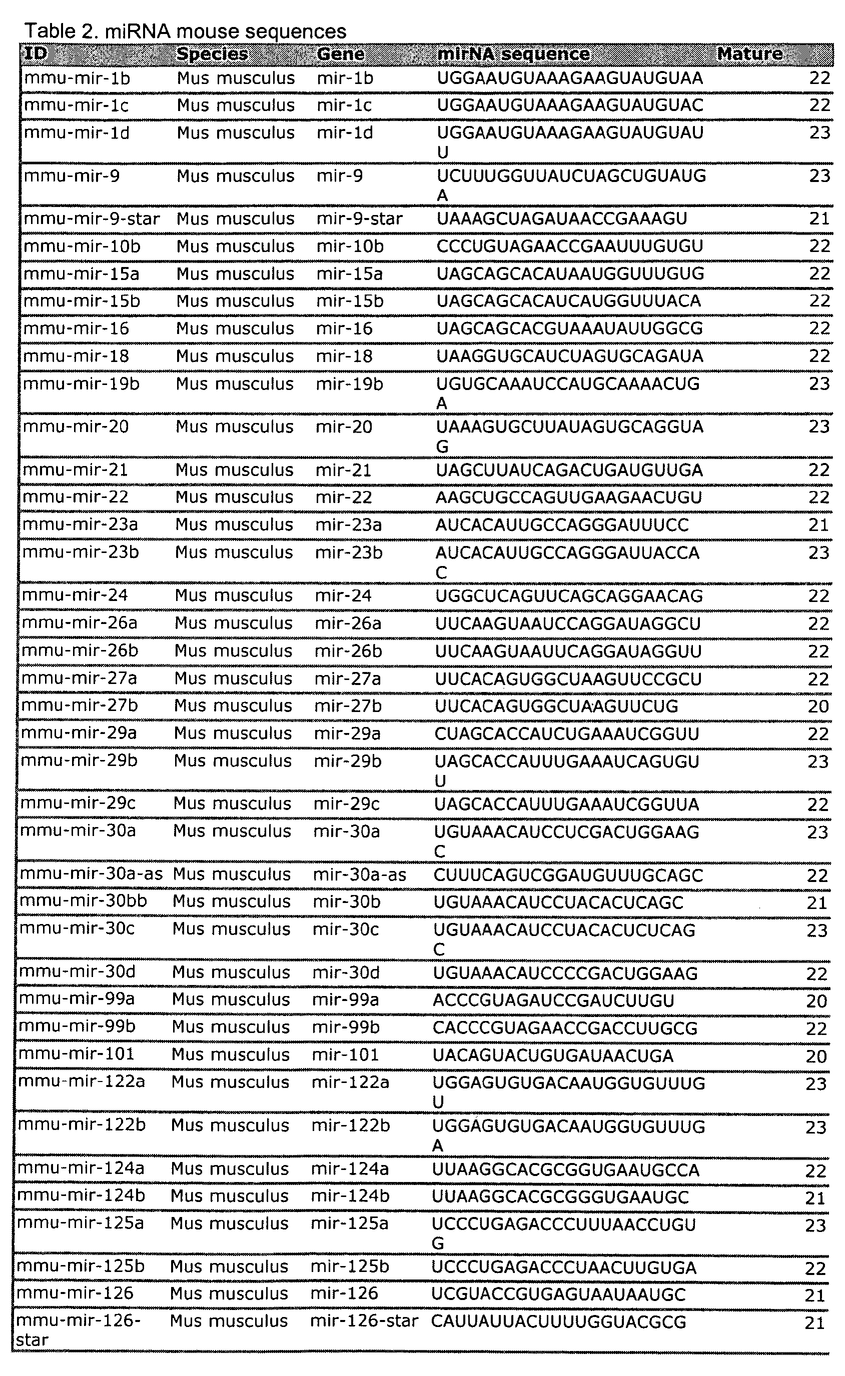

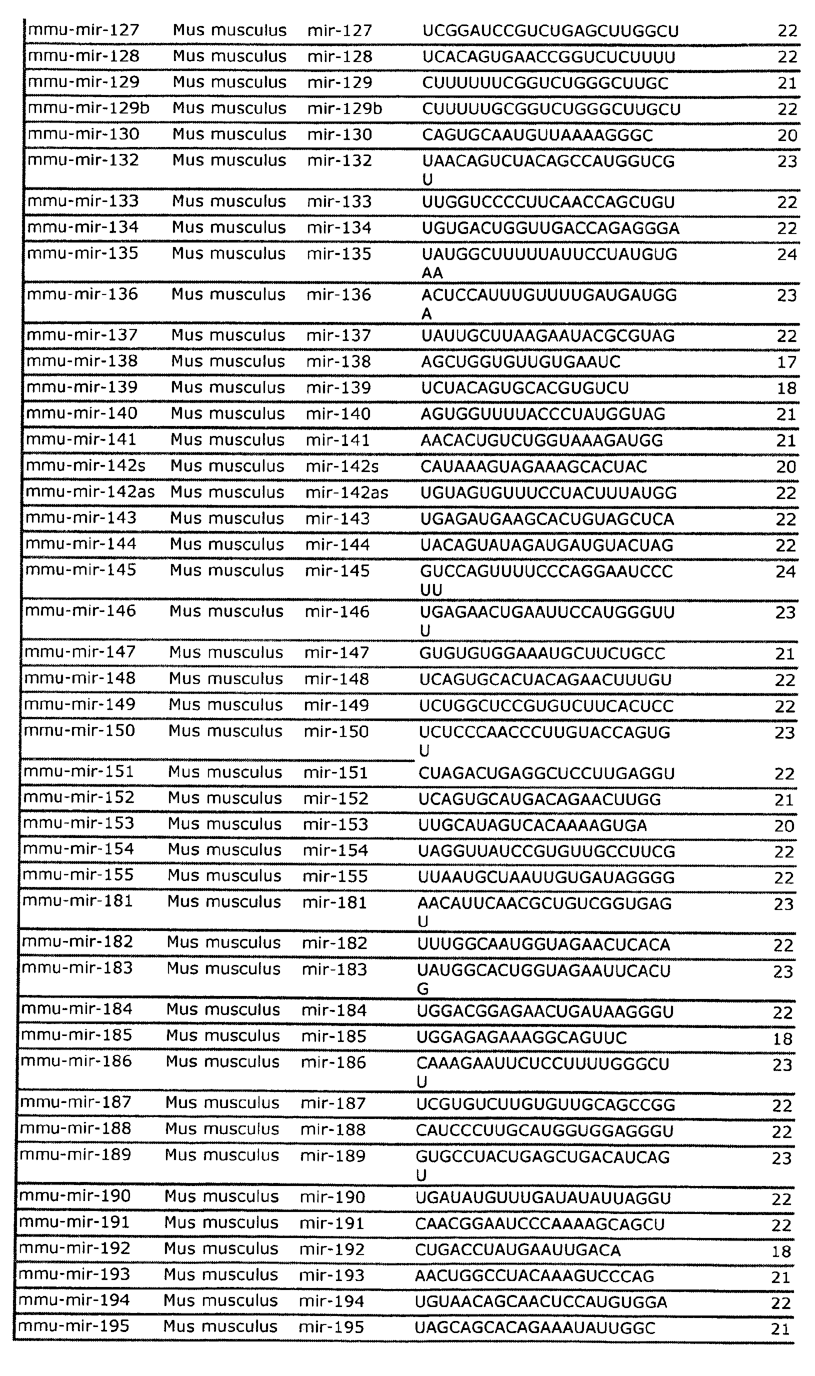

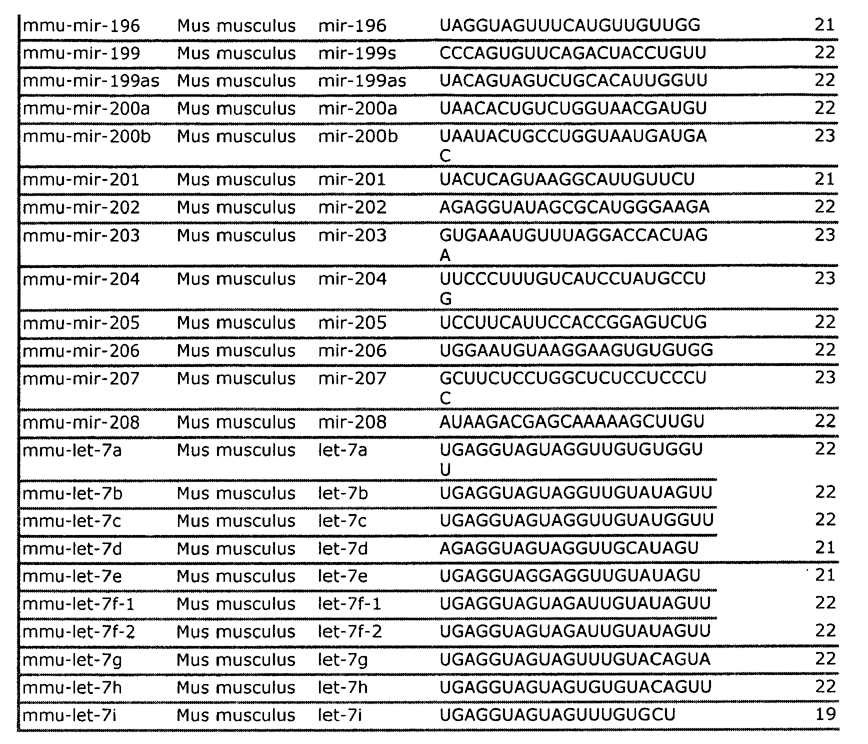

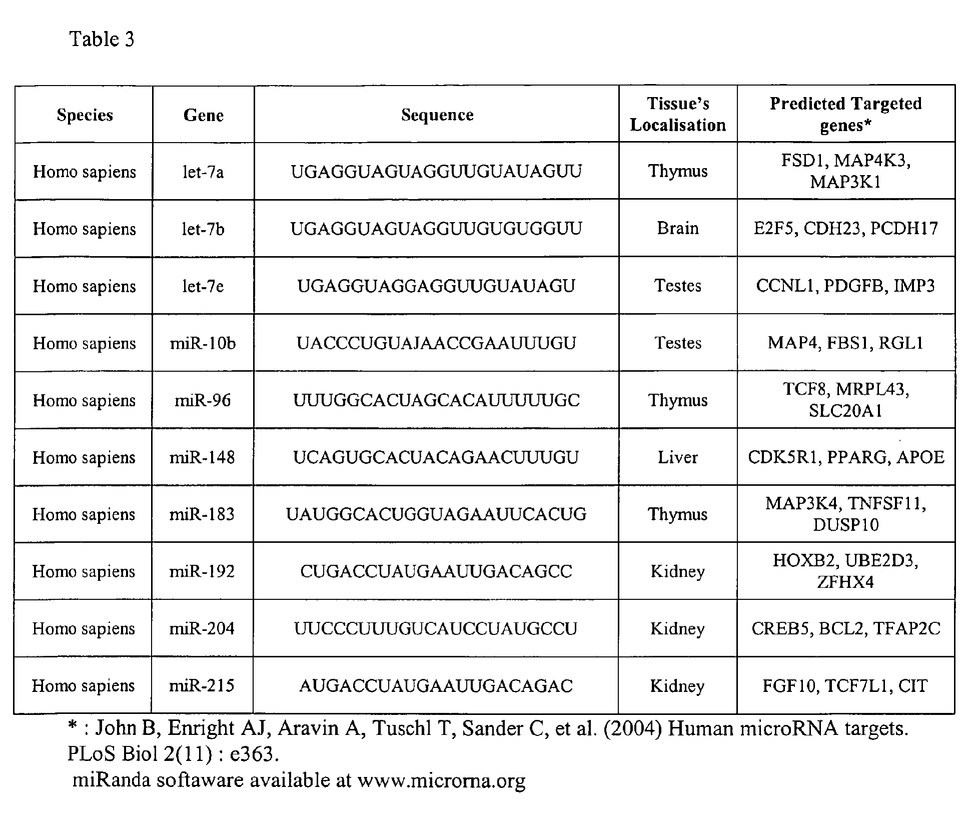

## Claims

1. A method for determining the RNAi mediated transcriptional regulation in a cell by the determination of a pattern of at least 3 miRNA detected simultaneously and quantified in the same cell extract, the method comprising the steps of:
(i) providing an array onto which capture probes, reflecting the genomic or transcriptional matter of a cell, are arranged on pre-determined locations thereof;
(ii) isolating a miRNA pool potentially present from a cell;
(iii) elongating or ligating said miRNAs into target labeled polynucleotides;
(iv) contacting said target labeled polynucleotides with the array under conditions allowing hybridization of the target labeled polynucleotides to complementary capture probes present on the array;
(v) detecting and quantifying a signal present on a specific location on the array;
wherein the detection of a pattern of at least 3 signals on the array reflects the pattern of miRNAs being involved in the RNAi mediated cellular transcriptional regulation.

2. The method of claim 1, wherein the RNAi mediated cellular transcriptional regulation provided by the detection and quantification of a pattern of miRNAs is correlated with the pattern of expression of
a) the regulated genes in the same sample; or
b) the miRNA targeted genes in the same sample; or
c) the genes having mRNA sequences complementary to the corresponding miRNA sequences in the same sample.

3. The method of claim 1, wherein the RNAi mediated cellular transcriptional regulation is related
a) to the development of an organism; or
b) to cell differentiation or stem cell maintenance; or
c) to cell proliferation; or
d) to cell death; or
e) to chromatin condensation; or
f) to cell transformation.

4. The method of claim 1, wherein the miRNA is incorporated into a labeled DNA-RNA sequence which is then detected on the array.

5. The method of claim 1, wherein elongation of the miRNA hybridized on its complementary bait sequence is effected with the Tth DNA polymerase 3, or with the AMV reverse transcriptase, or with the M-MLV reverse transcriptase.

6. The method of claim 1, wherein ligation of the miRNA hybridized on its complementary bait sequence is effected by ligation with an adjacent probe, preferably with a labeled adjacent probe.

7. The method of claim 6, wherein the adjacent probe is pre-hybridized with its complementary sequence before ligation with the miRNA.

8. The method of claim 6 - 7, wherein ligation of the miRNA with the adjacent probe is effected with the T4 RNA ligase.

9. The method of claim 4 - 5, wherein the elongation of the miRNA is effected on a sequence comprising three parts, wherein the 3' end is complementary of the miRNA, the middle part is specific of each bait, and the 5' end sequence is common to all baits.

10. The method of claim 9, wherein the elongated miRNAs are amplified.

11. The method of claim 10, wherein the amplification is performed after miRNA degradation using as matrix for the amplification a DNA/DNA hybrid complex.

12. The method of claim 9-11, wherein a primer complementary of the common sequence of the elongated DNA is provided for amplification.

13. The method of claim 12, wherein the amplification is performed with a DNA polymerase.

14. The method of claim 12, wherein the primer comprises a T7 promoter sequence for an RNA polymerase, or a Tag sequence, or wherein the primer is used for in vitro transcription with a RNA polymerase.

15. The method of claim 1, wherein the array comprises capture probes ranging from about 15 to about 1000 nucleotides, preferably from about 15 to about 200, or 15 to 100 nucleotides; and/or comprises 5-500 and preferably 5-5000 capture probes.

16. The method of claim 1, wherein the signals present on the array correspond to a pattern of at least 10 miRNAs, preferably at least 20 miRNAs.

17. The method of claims 15-16, wherein the capture probes have sequences which are identical for at least 10 to 1000 nucleotides to same part of the mRNA corresponding to the miRNA to be detected.

18. The method of claim 1, wherein at least 3 of the miRNA presented in Table 1 are simultaneously detected, or wherein at least 3 of the miRNA presented in Table 2 are simultaneously detected, or wherein at least 3 of the miRNA presented in Table 3 are simultaneously detected, wherein preferably each individually detected miRNA from Table 3 regulates one or several genes.

19. The method of claim 1, wherein the capture probes are able to detect both precursor and mature miRNA forms.

20. The method of claim 1, wherein the elongation of the miRNAs is effected on complementary bait sequences being circular and single stranded.

21. The method of claim 20, wherein the elongated miRNAs are amplified by rolling circle, or wherein the bait sequences being circular and single stranded are capture probes arranged on pre-determined locations of an array.

22. A kit for the determination of RNAi mediated cellular transcriptional regulation in a sample comprising an array comprising at least 3 capture probes being arranged on pre-determined locations and reflecting the genomic or transcriptional matter of a cell and optionally, buffers and labels, wherein preferably the first array is dedicated to the detection and of multiple miRNAs present and the second array is dedicated to the detection and quantification of the expression of the regulated genes in the same sample and optionally, buffers and labels, wherein the two arrays are preferably present on the same support, or
wherein the two arrays are present on the different supports.

23. A kit of claim 22, wherein the capture probes of the array for the detection of the miRNAs and of the mRNA are nucleotide sequences having part of their sequence identical to the mRNA.


### Annex to the application documents - subsequently filed sequences listing

SEQUENCE LISTING

<110>  Eppendorf Array Technologies

<120>  Method for the determination of cellular transcriptional regulation

<130>  80463 EP

<140>  EP 05 007 423.6
<141>  2005-04-05

<150>  US 10/818,956
<151>  2004-04-06

<160>  222

<170>  PatentIn version 3.1

<210>  1
<211>  21
<212>  RNA
<213>  Homo sapiens

<400>  1
uggaagacua gugauuuugu u                                                21

<210>  2
<211>  21
<212>  RNA
<213>  Homo sapiens

<400>  2
uggaagacua gugauuuugu u                                                21

<210>  3
<211>  21
<212>  RNA
<213>  Homo sapiens

<400>  3
uggaagacua gugauuuugu u                                                21

<210>  4
<211>  22
<212>  RNA
<213>  Homo sapiens

<400>  4
ugagguagua gauuguauag uu                                               22

<210>  5
<211>  22
<212>  RNA
<213>  Homo sapiens

<400>  5
ugagguagua gauuguauag uu                                               22

<210>  6
<211>  21
<212>  RNA
<213>  Homo sapiens

<400>  6
ugagguagga gguuguauag u                                                21


<210>  7
<211>  22
<212>  RNA
<213>  Homo sapiens

<400>  7
ugagguagua gguuguauag uu                                               22


<210>  8
<211>  22
<212>  RNA
<213>  Homo sapiens

<400>  8
ugagguagua gguuguauag uu                                               22


<210>  9
<211>  22
<212>  RNA
<213>  Homo sapiens

<400>  9
ugagguagua gguuguauag uu                                               22


<210>  10
<211>  22
<212>  RNA
<213>  Homo sapiens

<400>  10
ugagguagua gguugugugg uu                                               22


<210>  11
<211>  22
<212>  RNA
<213>  Homo sapiens

<400>  11
ugagguagua gguuguaugg uu                                               22


<210>  12
<211>  21
<212>  RNA
<213>  Homo sapiens

<400>  12
agagguagua gguugcauag u                                                21

<210>  13
<211>  23
<212>  RNA
<213>  Homo sapiens

<400>  13
uacccuguag auccgaauuu gug                                              23


<210>  14
<211>  22
<212>  RNA
<213>  Homo sapiens

<400>  14
uacccuguag aaccgaauuu gu                                               22


<210>  15
<211>  22
<212>  RNA
<213>  Homo sapiens

<400>  15
uagcagcaca uaaugguuug ug                                               22


<210>  16
<211>  22
<212>  RNA
<213>  Homo sapiens

<400>  16
uagcagcacg uaaauauugg cg                                               22


<210>  17
<211>  20
<212>  RNA
<213>  Homo sapiens

<400>  17
acugcaguga aggcacuugu                                                  20


<210>  18
<211>  22
<212>  RNA
<213>  Homo sapiens

<400>  18
uaaggugcau cuagugcaga ua                                               22


<210>  19
<211>  23
<212>  RNA
<213>  Homo sapiens

<400>  19
ugugcaaauc uaugcaaaac uga                                              23

<210>  20
<211>  23
<212>  RNA
<213>  Homo sapiens

<400>  20
ugugcaaauc caugcaaaac uga                                              23


<210>  21
<211>  23
<212>  RNA
<213>  Homo sapiens

<400>  21
ugugcaaauc caugcaaaac uga                                              23


<210>  22
<211>  22
<212>  RNA
<213>  Homo sapiens

<400>  22
uaaagugcuu auagugcagg ua                                               22


<210>  23
<211>  22
<212>  RNA
<213>  Homo sapiens

<400>  23
uagcuuauca gacugauguu ga                                               22


<210>  24
<211>  22
<212>  RNA
<213>  Homo sapiens

<400>  24
aagcugccag uugaagaacu gu                                               22


<210>  25
<211>  21
<212>  RNA
<213>  Homo sapiens

<400>  25
aucacauugc cagggauuuc c                                                21


<210>  26
<211>  22
<212>  RNA
<213>  Homo sapiens

<400>  26
uggcucaguu cagcaggaac ag                                               22

<210>  27
<211>  22
<212>  RNA
<213>  Homo sapiens

<400>  27
uggcucaguu cagcaggaac ag                                                22


<210>  28
<211>  22
<212>  RNA
<213>  Homo sapiens

<400>  28
cauugcacuu gucucggucu ga                                                22


<210>  29
<211>  22
<212>  RNA
<213>  Homo sapiens

<400>  29
uucaaguaau ccaggauagg cu                                                22


<210>  30
<211>  21
<212>  RNA
<213>  Homo sapiens

<400>  30
uucaaguaau ucaggauagg u                                                 21


<210>  31
<211>  22
<212>  RNA
<213>  Homo sapiens

<400>  31
uucacagugg cuaaguuccg cc                                                22


<210>  32
<211>  22
<212>  RNA
<213>  Homo sapiens

<400>  32
aaggagcuca cagucuauug ag                                                22


<210>  33
<211>  22
<212>  RNA
<213>  Homo sapiens

<400>  33
cuagcaccau cugaaaucgg uu                                                22

<210>  34
<211>  23
<212>  RNA
<213>  Homo sapiens

<400>  34
uguaaacauc cuacacucuc agc                                              23


<210>  35
<211>  22
<212>  RNA
<213>  Homo sapiens

<400>  35
uguaaacauc cccgacugga ag                                               22


<210>  36
<211>  23
<212>  RNA
<213>  Homo sapiens

<400>  36
uguaaacauc cucgacugga agc                                              23


<210>  37
<211>  22
<212>  RNA
<213>  Homo sapiens

<400>  37
cuuucagucg gauguuugca gc                                               22


<210>  38
<211>  21
<212>  RNA
<213>  Homo sapiens

<400>  38
ggcaagaugc uggcauagcu g                                                21


<210>  39
<211>  21
<212>  RNA
<213>  Homo sapiens

<400>  39
uauugcacau uacuaaguug c                                                21


<210>  40
<211>  19
<212>  RNA
<213>  Homo sapiens

<400>  40
gugcauugua guugcauug                                                   19

<210>  41
<211>  22
<212>  RNA
<213>  Homo sapiens

<400>  41
uggcaguguc uuagcugguu gu                                                22


<210>  42
<211>  24
<212>  RNA
<213>  Homo sapiens

<400>  42
caaagugcuu acagugcagg uagu                                              24


<210>  43
<211>  20
<212>  RNA
<213>  Homo sapiens

<400>  43
acugcaguga aggcacuugu                                                   20


<210>  44
<211>  22
<212>  RNA
<213>  Homo sapiens

<400>  44
uauugcacuu gucccggccu gu                                                22


<210>  45
<211>  22
<212>  RNA
<213>  Homo sapiens

<400>  45
uauugcacuu gucccggccu gu                                                22


<210>  46
<211>  22
<212>  RNA
<213>  Homo sapiens

<400>  46
aaagugcugu ucgugcaggu ag                                                22


<210>  47
<211>  22
<212>  RNA
<213>  Homo sapiens

<400>  47
aaagugcugu ucgugcaggu ag                                                22

<210>  48
<211>  22
<212>  RNA
<213>  Homo sapiens

<400>  48
uucaacgggu auuuauugag ca                                                22


<210>  49
<211>  22
<212>  RNA
<213>  Homo sapiens

<400>  49
uuuggcacua gcacauuuuu gc                                                22


<210>  50
<211>  22
<212>  RNA
<213>  Homo sapiens

<400>  50
ugagguagua aguuguauug uu                                                22


<210>  51
<211>  22
<212>  RNA
<213>  Homo sapiens

<400>  51
aacccguaga uccgaucuug ug                                                22


<210>  52
<211>  22
<212>  RNA
<213>  Homo sapiens

<400>  52
aacccguaga uccgaacuug ug                                                22


<210>  53
<211>  22
<212>  RNA
<213>  Homo sapiens

<400>  53
uacaguacug ugauaacuga ag                                                22


<210>  54
<211>  20
<212>  RNA
<213>  Homo sapiens

<400>  54
uagcaccauu ugaaaucagu                                                   20

<210>  55
<211>  20
<212>  RNA
<213>  Homo sapiens

<400>  55
uagcaccauu ugaaaucagu                                                    20


<210>  56
<211>  20
<212>  RNA
<213>  Homo sapiens

<400>  56
uagcaccauu ugaaaucagu                                                    20


<210>  57
<211>  23
<212>  RNA
<213>  Homo sapiens

<400>  57
agcaacauug uacagggcua uga                                                23


<210>  58
<211>  23
<212>  RNA
<213>  Homo sapiens

<400>  58
agcagcauug uacagggcua uga                                                23


<210>  59
<211>  22
<212>  RNA
<213>  Homo sapiens

<400>  59
ucaacaucag ucugauaagc ua                                                 22


<210>  60
<211>  20
<212>  RNA
<213>  Homo sapiens

<400>  60
ucaaaugcuc agacuccugu                                                    20


<210>  61
<211>  20
<212>  RNA
<213>  Homo sapiens

<400>  61
ucaaaugcuc agacuccugu                                                    20

<210>  62
<211>  24
<212>  RNA
<213>  Homo sapiens

<400>  62
aaaagugcuu acagugcagg uagc                                              24


<210>  63
<211>  23
<212>  RNA
<213>  Homo sapiens

<400>  63
agcagcauug uacagggcua uca                                               23


<210>  64
<211>  20
<212>  RNA
<213>  Homo sapiens

<400>  64
uuaaggcacg cggugaaugc                                                   20


<210>  65
<211>  18
<212>  RNA
<213>  Homo sapiens

<400>  65
ucuacagugc acgugucu                                                     18


<210>  66
<211>  20
<212>  RNA
<213>  Homo sapiens

<400>  66
   guguguggaa augcuucugc                                                  20


<210>  67
<211>  22
<212>  RNA
<213>  Homo sapiens

<400>  67
ucagugcacu acagaacuuu gu                                                22


<210>  68
<211>  22
<212>  RNA
<213>  Homo sapiens

<400>  68
aacauucaac cugucgguga gu                                                22

<210> 69
<211> 22
<212> RNA
<213> Homo sapiens

<400> 69
accaucgacc guugauugua cc 22


<210> 70
<211> 23
<212> RNA
<213> Homo sapiens

<400> 70
aacauucaac gcugucggug agu 23


<210> 71
<211> 21
<212> RNA
<213> Homo sapiens

<400> 71
ugguucuaga cuugccaacu a 21


<210> 72
<211> 23
<212> RNA
<213> Homo sapiens

<400> 72
uauggcacug guagaauuca cug 23


<210> 73
<211> 21
<212> RNA
<213> Homo sapiens

<400> 73
ucgugucuug uguugcagcc g 21


<210> 74
<211> 21
<212> RNA
<213> Homo sapiens

<400> 74
cugaccuaug aauugacagc c 21


<210> 75
<211> 22
<212> RNA
<213> Homo sapiens

<400> 75
uagguaguuu cauguuguug gg 22

<210>  76
<211>  22
<212>  RNA
<213>  Homo sapiens

<400>  76
uagguaguuu cauguuguug gg                                                22


<210>  77
<211>  21
<212>  RNA
<213>  Homo sapiens

<400>  77
uagguaguuu cauguuguug g                                                 21


<210>  78
<211>  22
<212>  RNA
<213>  Homo sapiens

<400>  78
uucaccaccu ucuccaccca gc                                                22


<210>  79
<211>  19
<212>  RNA
<213>  Homo sapiens

<400>  79
gguccagagg ggagauagg                                                    19


<210>  80
<211>  23
<212>  RNA
<213>  Homo sapiens

<400>  80
cccaguguuc agacuaccug uuc                                               23


<210>  81
<211>  23
<212>  RNA
<213>  Homo sapiens

<400>  81
cccaguguuu agacuaucug uuc                                               23


<210>  82
<211>  23
<212>  RNA
<213>  Homo sapiens

<400>  82
cccaguguuc agacuaccug uuc                                               23

<210> 83
<211> 22
<212> RNA
<213> Homo sapiens

<400> 83
cccaguguuc agacuaccug uu 22

<210> 84
<211> 24
<212> RNA
<213> Homo sapiens

<400> 84
cucuaauacu gccugguaau gaug 24

<210> 85
<211> 22
<212> RNA
<213> Homo sapiens

<400> 85
gugaaauguu uaggaccacu ag 22

<210> 86
<211> 22
<212> RNA
<213> Homo sapiens

<400> 86
uucccuuugu cauccuaugc cu 22

<210> 87
<211> 22
<212> RNA
<213> Homo sapiens

<400> 87
uccuucauuc caccggaguc ug 22

<210> 88
<211> 22
<212> RNA
<213> Homo sapiens

<400> 88
auaagacgag caaaaagcuu gu 22

<210> 89
<211> 21
<212> RNA
<213> Homo sapiens

<400> 89
cugugcgugu gacagcggcu g 21

<210>  90
<211>  22
<212>  RNA
<213>  Homo sapiens

<400>  90
uucccuuugu cauccuucgc cu                                                22


<210>  91
<211>  21
<212>  RNA
<213>  Homo sapiens

<400>  91
uaacagucuc cagucacggc c                                                 21


<210>  92
<211>  24
<212>  RNA
<213>  Homo sapiens

<400>  92
aacauucauu gcugucggug gguu                                              24


<210>  93
<211>  21
<212>  RNA
<213>  Homo sapiens

<400>  93
acagcaggca cagacaggca g                                                 21


<210>  94
<211>  21
<212>  RNA
<213>  Homo sapiens

<400>  94
augaccuaug aauugacaga c                                                 21


<210>  95
<211>  21
<212>  RNA
<213>  Homo sapiens

<400>  95
uaaucucagc uggcaacugu g                                                 21


<210>  96
<211>  24
<212>  RNA
<213>  Homo sapiens

<400>  96
uacugcauca ggaacugauu ggau                                              24

<210> 97
<211> 21
<212> RNA
<213> Homo sapiens

<400> 97
uugugcuuga ucuaaccaug u 21

<210> 98
<211> 21
<212> RNA
<213> Homo sapiens

<400> 98
uugugcuuga ucuaaccaug u 21

<210> 99
<211> 21
<212> RNA
<213> Homo sapiens

<400> 99
ugauugucca aacgcaauuc u 21

<210> 100
<211> 21
<212> RNA
<213> Homo sapiens

<400> 100
ccacaccgua ucugacacuu u 21

<210> 101
<211> 23
<212> RNA
<213> Homo sapiens

<400> 101
agcuacauug ucugcugggu uuc 23

<210> 102
<211> 24
<212> RNA
<213> Homo sapiens

<400> 102
agcuacaucu ggcuacuggg ucuc 24

<210> 103
<211> 21
<212> RNA
<213> Homo sapiens

<400> 103
ugucaguuug ucaaauaccc c 21

<210>  104
<211>  23
<212>  RNA
<213>  Homo sapiens

<400>  104
caagucacua gugguuccgu uua                                              23


<210>  105
<211>  22
<212>  RNA
<213>  Mus musculus

<400>  105
uggaauguaa agaaguaugu aa                                               22


<210>  106
<211>  22
<212>  RNA
<213>  Mus musculus

<400>  106
uggaauguaa agaaguaugu ac                                               22


<210>  107
<211>  23
<212>  RNA
<213>  Mus musculus

<400>  107
uggaauguaa agaaguaugu auu                                              23


<210>  108
<211>  23
<212>  RNA
<213>  Mus musculus

<400>  108
ucuuugguua ucuagcugua uga                                              23


<210>  109
<211>  21
<212>  RNA
<213>  Mus musculus

<400>  109
uaaagcuaga uaaccgaaag u                                                21


<210>  110
<211>  22
<212>  RNA
<213>  Mus musculus

<400>  110
cccuguagaa ccgaauuugu gu                                               22

<210> 111
<211> 22
<212> RNA
<213> Mus musculus

<400> 111
uagcagcaca uaaugguuug ug 22


<210> 112
<211> 22
<212> RNA
<213> Mus musculus

<400> 112
uagcagcaca ucaugguuua ca 22


<210> 113
<211> 22
<212> RNA
<213> Mus musculus

<400> 113
uagcagcacg uaaauauugg cg 22


<210> 114
<211> 22
<212> RNA
<213> Mus musculus

<400> 114
uaaggugcau cuagugcaga ua 22


<210> 115
<211> 23
<212> RNA
<213> Mus musculus

<400> 115
ugugcaaauc caugcaaaac uga 23


<210> 116
<211> 23
<212> RNA
<213> Mus musculus

<400> 116
uaaagugcuu auagugcagg uag 23


<210> 117
<211> 22
<212> RNA
<213> Mus musculus

<400> 117
uagcuuauca gacugauguu ga 22

<210> 118
<211> 22
<212> RNA
<213> Mus musculus

<400> 118
aagcugccag uugaagaacu gu 22


<210> 119
<211> 21
<212> RNA
<213> Mus musculus

<400> 119
aucacauugc cagggauuuc c 21


<210> 120
<211> 23
<212> RNA
<213> Mus musculus

<400> 120
aucacauugc cagggauuac cac 23


<210> 121
<211> 22
<212> RNA
<213> Mus musculus

<400> 121
uggcucaguu cagcaggaac ag 22


<210> 122
<211> 22
<212> RNA
<213> Mus musculus

<400> 122
uucaaguaau ccaggauagg cu 22


<210> 123
<211> 22
<212> RNA
<213> Mus musculus

<400> 123
uucaaguaau ucaggauagg uu 22


<210> 124
<211> 22
<212> RNA
<213> Mus musculus

<400> 124
uucacagugg cuaaguuccg cu 22

<210>  125
<211>  20
<212>  RNA
<213>  Mus musculus

<400>  125
uucacagugg cuaaguucug                                                    20


<210>  126
<211>  22
<212>  RNA
<213>  Mus musculus

<400>  126
cuagcaccau cugaaaucgg uu                                                 22


<210>  127
<211>  23
<212>  RNA
<213>  Mus musculus

<400>  127
uagcaccauu ugaaaucagu guu                                                23


<210>  128
<211>  22
<212>  RNA
<213>  Mus musculus

<400>  128
uagcaccauu ugaaaucggu ua                                                 22


<210>  129
<211>  23
<212>  RNA
<213>  Mus musculus

<400>  129
uguaaacauc cucgacugga agc                                                23


<210>  130
<211>  22
<212>  RNA
<213>  Mus musculus

<400>  130
cuuucagucg gauguuugca gc                                                 22


<210>  131
<211>  21
<212>  RNA
<213>  Mus musculus

<400>  131
uguaaacauc cuacacucag c                                                  21

<210> 132
<211> 23
<212> RNA
<213> Mus musculus

<400> 132
uguaaacauc cuacacucuc agc 23

<210> 133
<211> 22
<212> RNA
<213> Mus musculus

<400> 133
uguaaacauc cccgacugga ag 22

<210> 134
<211> 20
<212> RNA
<213> Mus musculus

<400> 134
acccguagau ccgaucuugu 20

<210> 135
<211> 22
<212> RNA
<213> Mus musculus

<400> 135
cacccguaga accgaccuug cg 22

<210> 136
<211> 20
<212> RNA
<213> Mus musculus

<400> 136
uacaguacug ugauaacuga 20

<210> 137
<211> 23
<212> RNA
<213> Mus musculus

<400> 137
uggaguguga caaugguguu ugu 23

<210> 138
<211> 23
<212> RNA
<213> Mus musculus

<400> 138
uggaguguga caaugguguu uga 23

<210> 139
<211> 22
<212> RNA
<213> Mus musculus

<400> 139
uuaaggcacg cggugaaugc ca 22


<210> 140
<211> 21
<212> RNA
<213> Mus musculus

<400> 140
uuaaggcacg cgggugaaug c 21


<210> 141
<211> 23
<212> RNA
<213> Mus musculus

<400> 141
ucccugagac ccuuuaaccu gug 23


<210> 142
<211> 22
<212> RNA
<213> Mus musculus

<400> 142
ucccugagac ccuaacuugu ga 22


<210> 143
<211> 21
<212> RNA
<213> Mus musculus

<400> 143
ucguaccgug aguaauaaug c 21


<210> 144
<211> 21
<212> RNA
<213> Mus musculus

<400> 144
cauuauuacu uuugguacgc g 21


<210> 145
<211> 22
<212> RNA
<213> Mus musculus

<400> 145
ucggauccgu cugagcuugg cu 22

<210>  146
<211>  22
<212>  RNA
<213>  Mus musculus

<400>  146
ucacagugaa ccggucucuu uu                                                22


<210>  147
<211>  21
<212>  RNA
<213>  Mus musculus

<400>  147
cuuuuuucgg ucugggcuug c                                                 21


<210>  148
<211>  22
<212>  RNA
<213>  Mus musculus

<400>  148
cuuuuugcgg ucugggcuug cu                                                22


<210>  149
<211>  20
<212>  RNA
<213>  Mus musculus

<400>  149
cagugcaaug uuaaaagggc                                                   20


<210>  150
<211>  23
<212>  RNA
<213>  Mus musculus

<400>  150
uaacagucua cagccauggu cgu                                               23


<210>  151
<211>  22
<212>  RNA
<213>  Mus musculus

<400>  151
uugguccccu ucaaccagcu gu                                                22


<210>  152
<211>  22
<212>  RNA
<213>  Mus musculus

<400>  152
ugugacuggu ugaccagagg ga                                                22

<210>  153
<211>  24
<212>  RNA
<213>  Mus musculus

<400>  153
uauggcuuuu uauuccuaug ugaa                                              24


<210>  154
<211>  23
<212>  RNA
<213>  Mus musculus

<400>  154
acuccauuug uuuugaugau gga                                               23


<210>  155
<211>  22
<212>  RNA
<213>  Mus musculus

<400>  155
uauugcuuaa gaauacgcgu ag                                                22


<210>  156
<211>  17
<212>  RNA
<213>  Mus musculus

<400>  156
agcugguguu gugaauc                                                      17


<210>  157
<211>  18
<212>  RNA
<213>  Mus musculus

<400>  157
ucuacagugc acgugucu                                                     18


<210>  158
<211>  21
<212>  RNA
<213>  Mus musculus

<400>  158
agugguuuua cccuauggua g                                                 21


<210>  159
<211>  21
<212>  RNA
<213>  Mus musculus

<400>  159
aacacugucu gguaaagaug g                                                 21

<210> 160
<211> 20
<212> RNA
<213> Mus musculus

<400> 160
cauaaaguag aaagcacuac 20


<210> 161
<211> 22
<212> RNA
<213> Mus musculus

<400> 161
uguaguguuu ccuacuuuau gg 22


<210> 162
<211> 22
<212> RNA
<213> Mus musculus

<400> 162
ugagaugaag cacuguagcu ca 22


<210> 163
<211> 22
<212> RNA
<213> Mus musculus

<400> 163
uacaguauag augauguacu ag 22


<210> 164
<211> 24
<212> RNA
<213> Mus musculus

<400> 164
guccaguuuu cccaggaauc ccuu 24


<210> 165
<211> 23
<212> RNA
<213> Mus musculus

<400> 165
ugagaacuga auuccauggg uuu 23


<210> 166
<211> 21
<212> RNA
<213> Mus musculus

<400> 166
guguguggaa augcuucugc c 21

<210>  167
<211>  22
<212>  RNA
<213>  Mus musculus

<400>  167
ucagugcacu acagaacuuu gu                                                22


<210>  168
<211>  22
<212>  RNA
<213>  Mus musculus

<400>  168
ucuggcuccg ugucuucacu cc                                                22


<210>  169
<211>  23
<212>  RNA
<213>  Mus musculus

<400>  169
ucucccaacc cuuguaccag ugu                                               23


<210>  170
<211>  22
<212>  RNA
<213>  Mus musculus

<400>  170
cuagacugag gcuccuugag gu                                                22


<210>  171
<211>  21
<212>  RNA
<213>  Mus musculus

<400>  171
ucagugcaug acagaacuug g                                                 21


<210>  172
<211>  20
<212>  RNA
<213>  Mus musculus

<400>  172
uugcauaguc acaaaaguga                                                   20


<210>  173
<211>  22
<212>  RNA
<213>  Mus musculus

<400>  173
uagguuaucc guguugccuu cg                                                22

<210>  174
<211>  22
<212>  RNA
<213>  Mus musculus

<400>  174
uuaaugcuaa uugugauagg gg                                                22


<210>  175
<211>  23
<212>  RNA
<213>  Mus musculus

<400>  175
aacauucaac gcugucggug agu                                               23


<210>  176
<211>  22
<212>  RNA
<213>  Mus musculus

<400>  176
uuuggcaaug guagaacuca ca                                                22


<210>  177
<211>  23
<212>  RNA
<213>  Mus musculus

<400>  177
uauggcacug guagaauuca cug                                               23


<210>  178
<211>  22
<212>  RNA
<213>  Mus musculus

<400>  178
uggacggaga acugauaagg gu                                                22


<210>  179
<211>  18
<212>  RNA
<213>  Mus musculus

<400>  179
uggagagaaa ggcaguuc                                                     18


<210>  180
<211>  23
<212>  RNA
<213>  Mus musculus

<400>  180
caaagaauuc uccuuuuggg cuu                                               23

<210>  181
<211>  22
<212>  RNA
<213>  Mus musculus

<400>  181
ucgugucuug uguugcagcc gg                                                22


<210>  182
<211>  22
<212>  RNA
<213>  Mus musculus

<400>  182
caucccuugc augguggagg gu                                                22


<210>  183
<211>  23
<212>  RNA
<213>  Mus musculus

<400>  183
gugccuacug agcugacauc agu                                               23


<210>  184
<211>  22
<212>  RNA
<213>  Mus musculus

<400>  184
ugauauguuu gauauauuag gu                                                22


<210>  185
<211>  22
<212>  RNA
<213>  Mus musculus

<400>  185
caacggaauc ccaaaagcag cu                                                22


<210>  186
<211>  18
<212>  RNA
<213>  Mus musculus

<400>  186
cugaccuaug aauugaca                                                     18


<210>  187
<211>  21
<212>  RNA
<213>  Mus musculus

<400>  187
   aacuggccua caaaguccca g                                                21

<210>  188
<211>  22
<212>  RNA
<213>  Mus musculus

<400>  188
uguaacagca acuccaugug ga                                                22


<210>  189
<211>  21
<212>  RNA
<213>  Mus musculus

<400>  189
uagcagcaca gaaauauugg c                                                 21


<210>  190
<211>  21
<212>  RNA
<213>  Mus musculus

<400>  190
uagguaguuu cauguuguug g                                                 21


<210>  191
<211>  22
<212>  RNA
<213>  Mus musculus

<400>  191
cccaguguuc agacuaccug uu                                                22


<210>  192
<211>  22
<212>  RNA
<213>  Mus musculus

<400>  192
uacaguaguc ugcacauugg uu                                                22


<210>  193
<211>  22
<212>  RNA
<213>  Mus musculus

<400>  193
uaacacuguc ugguaacgau gu                                                22


<210>  194
<211>  23
<212>  RNA
<213>  Mus musculus

<400>  194
uaauacugcc ugguaaugau gac                                               23

<210>  195
<211>  21
<212>  RNA
<213>  Mus musculus

<400>  195
uacucaguaa ggcauuguuc u                                                21


<210>  196
<211>  22
<212>  RNA
<213>  Mus musculus

<400>  196
agagguauag cgcaugggaa ga                                               22


<210>  197
<211>  23
<212>  RNA
<213>  Mus musculus

<400>  197
gugaaauguu uaggaccacu aga                                              23


<210>  198
<211>  23
<212>  RNA
<213>  Mus musculus

<400>  198
uucccuuugu cauccuaugc cug                                              23


<210>  199
<211>  22
<212>  RNA
<213>  Mus musculus

<400>  199
uccuucauuc caccggaguc ug                                               22


<210>  200
<211>  22
<212>  RNA
<213>  Mus musculus

<400>  200
uggaauguaa ggaagugugu gg                                               22


<210>  201
<211>  23
<212>  RNA
<213>  Mus musculus

<400>  201
gcuucuccug gcucuccucc cuc                                              23

<210>  202
<211>  22
<212>  RNA
<213>  Mus musculus

<400>  202
auaagacgag caaaaagcuu gu                                                22


<210>  203
<211>  22
<212>  RNA
<213>  Mus musculus

<400>  203
ugagguagua gguugugugg uu                                                22


<210>  204
<211>  22
<212>  RNA
<213>  Mus musculus

<400>  204
ugagguagua gguuguauag uu                                                22


<210>  205
<211>  22
<212>  RNA
<213>  Mus musculus

<400>  205
ugagguagua gguuguaugg uu                                                22


<210>  206
<211>  21
<212>  RNA
<213>  Mus musculus

<400>  206
agagguagua gguugcauag u                                                 21


<210>  207
<211>  21
<212>  RNA
<213>  Mus musculus

<400>  207
ugagguagga gguuguauag u                                                 21


<210>  208
<211>  22
<212>  RNA
<213>  Mus musculus

<400>  208
ugagguagua gauuguauag uu                                                22

<210>  209
<211>  22
<212>  RNA
<213>  Mus musculus

<400>  209
ugagguagua gauuguauag uu                                                22

<210>  210
<211>  22
<212>  RNA
<213>  Mus musculus

<400>  210
ugagguagua guuuguacag ua                                                22

<210>  211
<211>  22
<212>  RNA
<213>  Mus musculus

<400>  211
ugagguagua guguguacag uu                                                22

<210>  212
<211>  19
<212>  RNA
<213>  Mus musculus

<400>  212
ugagguagua guuugugcu                                                    19

<210>  213
<211>  22
<212>  RNA
<213>  Homo sapiens

<400>  213
ugagguagua gguuguauag uu                                                22

<210>  214
<211>  22
<212>  RNA
<213>  Homo sapiens

<400>  214
ugagguagua gguugugugg uu                                                22

<210>  215
<211>  21
<212>  RNA
<213>  Homo sapiens

<400>  215
ugagguagga gguuguauag u                                                 21

<210>  216
<211>  22
<212>  RNA
<213>  Homo sapiens

<220>
<221>  misc_feature
<222>  (10)..(10)
<223>  any, unknown or other


<400>  216
uacccuguan aaccgaauuu gu                                                22


<210>  217
<211>  22
<212>  RNA
<213>  Homo sapiens

<400>  217
uuuggcacua gcacauuuuu gc                                                22


<210>  218
<211>  22
<212>  RNA
<213>  Homo sapiens

<400>  218
ucagugcacu acagaacuuu gu                                                22


<210>  219
<211>  23
<212>  RNA
<213>  Homo sapiens

<400>  219
uauggcacug guagaauuca cug                                               23


<210>  220
<211>  21
<212>  RNA
<213>  Homo sapiens

<400>  220
cugaccuaug aauugacagc c                                                 21


<210>  221
<211>  22
<212>  RNA
<213>  Homo sapiens

<400>  221
uucccuuugu cauccuaugc cu                                                22


<210>  222
<211>  21
<212>  RNA

<213>  Homo sapiens

<400>  222
augaccuaug aauugacaga c                                                21